(19) **Europäisches Patentamt**
**European Patent Office**
**Office européen des brevets**

(11) **EP 4 664 487 A1**

(12) **EUROPEAN PATENT APPLICATION**

(43) Date of publication:
**17.12.2025 Bulletin 2025/51**

(21) Application number: **25177169.7**

(22) Date of filing: **19.05.2025**

(51) International Patent Classification (IPC):
**G16H 50/70** (2018.01)

(52) Cooperative Patent Classification (CPC):
**G16H 50/70; G16H 50/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **10.06.2024 US 202418738722**

(71) Applicant: **GE Precision Healthcare LLC**
**Waukesha, WI 53188 (US)**

(72) Inventors:
• **PARDASANI, Rohit**
**560066 Bengaluru (IN)**
• **AJITH, Siddharth**
**560066 Bengaluru (IN)**

(74) Representative: **Kilburn & Strode LLP**
**Lacon London**
**84 Theobalds Road**
**Holborn**
**London WC1X 8NL (GB)**

(54) **CLINICAL PROTOCOL-BASED FEDERATED LEARNING**

(57)    One or more systems, devices, computer program products and/or computer-implemented methods of use provided herein relate to a clinical protocol-based federated learning process. For example, a system can comprise a memory that can store computer executable components. The system can further comprise a processor that can execute at least one of the computer executable components that can select, according to a selection criterion applicable to at least one medical facility, a first artificial intelligence (AI) model from a repository comprising a plurality of AI models, deploy the first AI model at the at least one medical facility, access feedback comprising updated parameters of the first AI model generated at the at least one medical facility, and further deploy a second AI model based on the updated parameters and a clinical protocol employed by the at least one medical facility.

**FIG. 1**

**Description**

BACKGROUND

The subject disclosure relates to machine learning and, more specifically, to a clinical protocol-based federated learning process.

SUMMARY

The following presents a summary to provide a basic understanding of one or more embodiments described herein. This summary is not intended to identify key or critical elements, delineate scope of particular embodiments or scope of claims. Its sole purpose is to present concepts in a simplified form as a prelude to the more detailed description that is presented later. In one or more embodiments described herein, systems, computer-implemented methods, apparatus and/or computer program products that enable a clinical protocol-based federated learning process are discussed.

According to an embodiment, a system is provided. The system can comprise a memory that can store computer executable components. The system can further comprise a processor that can execute at least one of the computer executable components. The at least one of the computer executable components can select, according to a selection criterion applicable to at least one medical facility, a first artificial intelligence (AI) model from a repository comprising a plurality of AI models. The at least one of the computer executable components can deploy the first AI model at the at least one medical facility. The at least one of the computer executable components can access feedback comprising updated parameters of the first AI model generated at the at least one medical facility. The at least one of the computer executable components can further deploy a second AI model based on the updated parameters and a clinical protocol employed by the at least one medical facility.

According to another embodiment, a computer-implemented method is provided. The computer-implemented method can comprise selecting, by a system operatively coupled to a processor, according to a selection criterion applicable to at least one medical facility, a first AI model from a repository comprising a plurality of AI models. The computer-implemented method can further comprise deploying, by the system, the first AI model at the at least one medical facility. The computer-implemented method can further comprise accessing, by the system, feedback comprising updated parameters of the first AI model generated at the at least one medical facility. The computer-implemented method can further comprise deploying, by the system, a second AI model based on the updated parameters and a clinical protocol employed by the at least one medical facility.

According to yet another embodiment, a computer program product is provided. The computer program product can comprise a non-transitory computer readable memory having program instructions embodied therewith. The program instructions can be executable by a processor to cause the processor to select, according to a selection criterion applicable to at least one medical facility, a first AI model from a repository comprising a plurality of AI models. The program instructions can be further executable by the processor to cause the processor to deploy the first AI model at the at least one medical facility. The program instructions can be further executable by the processor to cause the processor to access feedback comprising updated parameters of the first AI model generated at the at least one medical facility. The program instructions can be further executable by a processor to cause the processor to deploy a second AI model based on the updated parameters and a clinical protocol employed by the at least one medical facility.

BRIEF DESCRIPTION OF THE DRAWINGS

One or more embodiments are described below in the Detailed Description section with reference to the following drawings:

FIG. 1 illustrates a diagram of an example, non-limiting process applicable to federated learning.
FIG. 2A illustrates a block diagram of an example, non-limiting system that can generate one or more AI models directed to different clinical protocols in accordance with one or more embodiments described herein.
FIG. 2B illustrates a block diagram of an example, non-limiting system that can generate one or more AI models directed to different clinical protocols in accordance with one or more embodiments described herein.
FIG. 3 illustrates diagrams of example, non-limiting methods applicable to federated learning.
FIG. 4 illustrates a flow diagram of an example, non-limiting method that can be employed as part of a protocol-based federated learning process in accordance with one or more embodiments described herein.
FIG. 5 illustrates another flow diagram of an example, non-limiting method that can be employed as part of a protocol-based federated learning process in accordance with one or more embodiments described herein.
FIG. 6 illustrates a diagram of an example, non-limiting graph that shows data that can be employed to train an AI model based on a clinical protocol in accordance with one or more embodiments described herein.

FIG. 7 illustrates diagrams of example, non-limiting graphs that show data that can be employed to train different AI models directed to different respective clinical protocols in accordance with one or more embodiments described herein.

FIG. 8A illustrates diagrams of example, non-limiting graphs that show training curves for different AI model directed to different respective clinical protocols in accordance with one or more embodiments described herein.

FIG. 8B illustrates a flow diagram of an example, non-limiting method to train AI models directed to different respective clinical protocols in accordance with one or more embodiments described herein.

FIG. 9 illustrates diagrams of example, non-limiting graph sets that show AUC curves for different AI models directed to different respective clinical protocols in accordance with one or more embodiments described herein.

FIG. 10 illustrates a process flow diagram of an example, non-limiting pipeline that can be employed to generate one or more AI models directed to different clinical protocols in accordance with one or more embodiments described herein.

FIG. 11 illustrates a flow diagram of an example, non-limiting method that can generate one or more AI models directed to different clinical protocols in accordance with one or more embodiments described herein.

FIG. 12 illustrates a block diagram of an example, non-limiting operating environment in which one or more embodiments described herein can be facilitated.

FIG. 13 illustrates an example networking environment operable to execute various implementations described herein.

## DETAILED DESCRIPTION

The following detailed description is merely illustrative and is not intended to limit embodiments and/or application or uses of embodiments. Furthermore, there is no intention to be bound by any expressed or implied information presented in the preceding Background or Summary sections, or in the Detailed Description section.

One or more embodiments are now described with reference to the drawings, wherein like referenced numerals are used to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

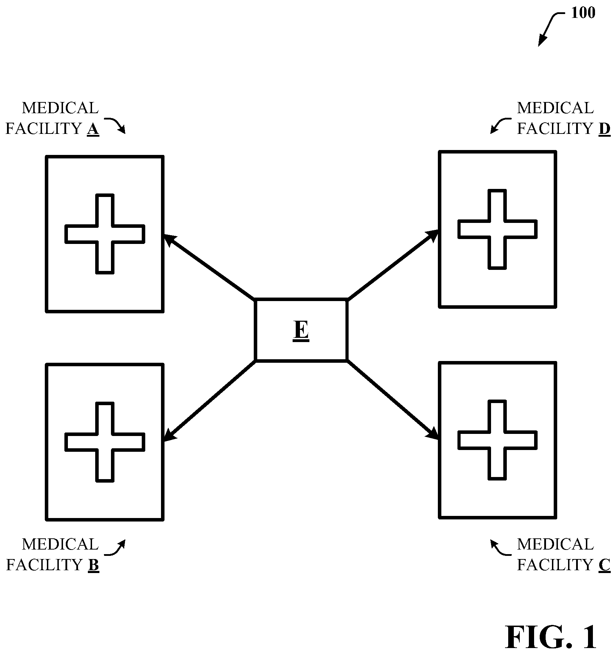

Classical or traditional federated learning involves a central AI model and multiple local AI models deployed at different local sites or locations. In non-federated learning, local data (e.g., patient data or other types of data related to individuals or locations) specific to different locations (e.g., medical facilities A, B, C and D) can be collected, and a central AI model can be trained at a central location by a central server for inferencing certain outcomes based on the local data. However, local sites such as medical facilities or other non-healthcare related sites can have reservations in sharing their local data with third parties because such local data can comprise confidential information about individuals such as patient, customers, etc. Through traditional federated learning, the local sites can share information that is relevant to the training of the central AI model, without disclosing confidential information, such that the central AI model does not have access to the local data that is processed by the local models. For example, as illustrated by non-limiting system 100 in FIG. 1, medical facilities A, B, C and D can train local AI models in silos based on a central AI model that is deployed at the medical facilities by a central server, E. For example, medical facility A can employ the architecture of the central AI model to train and generate a local AI model (e.g., model A) based on local data that is specific to medical facility A. The training can generate new weights and parameters for model A, and medical facility A can iteratively share the new weights and parameters with the central server, E. Similarly, medical facility B can employ the architecture of the central AI model to generate a local AI model (e.g., model B) based on local data that is specific to medical facility B, and medical facility B can iteratively share new weights and parameters of model B with the central server, E. As such, the weights and parameters of $N$ local AI models can be respectively shared by N medical facilities with the central server.

By employing the weights and parameters of $N$ local AI models, the central AI model can be iteratively trained by the central server, E, to update the parameters of the central AI model according to the local data generated at the N medical facilities, without the local data being disclosed to the central server. Training the central AI model can involve an aggregation of all the weights and parameters generated at the different local sites under the assumption that each local AI model is continuously improving by certain proportions based on the local data processed by each local AI model at the respective local sites. By aggregating the weights and parameters of the local AI models, the central AI model can be trained on the local data from different local sites, and the central server, E, can deploy at each local site, an updated central AI model. The updated central AI model can be a generalized AI model with weights and parameters that can be applicable to multiple different local sites. In this regard, the architecture of the central AI model is identical to the respective architectures of the local AI models so that the weights and parameters of the local AI models can match those of the central AI model, and weights can be transferred between the central and local AI models. In some cases, the different local sites can maintain their own local AI models separately in addition to the central AI model.

However, different local sites can employ different protocols. For example, different medical facilities can follow different clinical protocols, and a single central AI model is not applicable to all medical facilities because a central AI model trained on local data from medical facilities following different respective clinical protocols can generate inferences based on an average of thresholds associated with the different respective clinical protocols. As a result, the central AI model can exhibit an undesirable performance at each medical facility. Existing approaches in this regard are not customized to clinical protocols and are fixed and consistent across medical facilities. Such existing approaches also do not define any model selection process to determine which AI model to select and iterate upon. Thus, a more nuanced approach to federated learning can be desirable.

Embodiments described herein include systems, computer-implemented methods, and computer program products that can generate different AI models directed to different clinical protocols followed by medical facilities. For example, in an embodiment, a central server can employ a machine learning model to generate different AI models directed to different respective clinical protocols, and the central server can store the different AI models in a repository. Each AI model directed to a clinical protocol can be deployed to generate inferences at one or more medical facilities that follow the same clinical protocol. For example, an AI model can be trained to predict fetal heart rate (FHR) accelerations according to a clinical protocol, such that the AI model can classify only the FHR values that meet the criteria defined by the clinical protocol, as FHR accelerations.

In an embodiment, when a new medical facility signs up with the central server, the machine learning model can select from the repository, an AI model that is directed to the clinical protocol followed by the medical facility. The machine learning model can rely on properties such as the clinical protocol, demographics, geographical locations, etc. associated with the medical facility to select the AI model. For example, in an embodiment, the machine learning model can tag each AI model stored in the repository with tags that identify the clinical protocol that the AI model is trained for, demographic information associated with data employed to train the AI model, etc. The machine learning model can acquire information about identical properties associated with the medical facility and match the properties associated with the medical facility with the tags assigned to the AI models in the repository. In another embodiment, each AI model can be associated with a property table that lists the properties associated with the AI model, such as the clinical protocol that the AI model is trained for, demographic information associated with data employed to train the AI model, etc. The machine learning model can acquire a similar property table for the medical facility and compare the categories in the property table to identical categories in respective property tables associated with the AI models in the repository to select the AI model to be deployed at the medical facility. If the clinical protocol followed by the medical facility is known and an AI model directed to the clinical protocol does not exist in the repository, the machine learning model can select an AI model directed to a different clinical protocol that is closest to the clinical protocol followed by the medical facility. For example, the machine learning model can select an AI model according to a performance metric of the AI model for the clinical protocol.

After selecting the AI model, the machine learning model can deploy the AI model at the medical facility where the AI model can be employed to generate inferences on local data, and clinical feedback and labels assigned to the inferences by an entity (e.g., hardware, software, neural network, AI, machine and/or user) can be employed in conjunction with the local data to further train the AI model. The clinical feedback and labels and the updated weights and gradients generated as a result of the training can be accessed by the machine learning model as feedback. In an embodiment, the machine learning model can employ the feedback to retrain the AI model to generate and deploy a new AI model at the medical facility. In another embodiment, the machine learning model can employ the feedback to train an untrained AI model to generate and deploy a new AI model, for example, if an AI model directed to the clinical protocol does not exist in the repository. In yet another embodiment, the machine learning model can employ the feedback to select and deploy a different existing AI model from the repository if the clinical protocol followed by the medical facility was initially unknown. The AI models deployed at the medical facility and any other medical facilities serviced by the central server can be iteratively trained and updated as part of a protocol-based federated learning process.

It should be noted that the embodiments of the present disclosure are not limited to applications in healthcare. The various embodiments herein can be applied to any industry where data privacy is an issue and protocols are enforced. The various embodiments herein can also be employed to achieve standardization of protocols when protocols are fuzzy or undefined, or to define a protocol when a protocol does not exist.

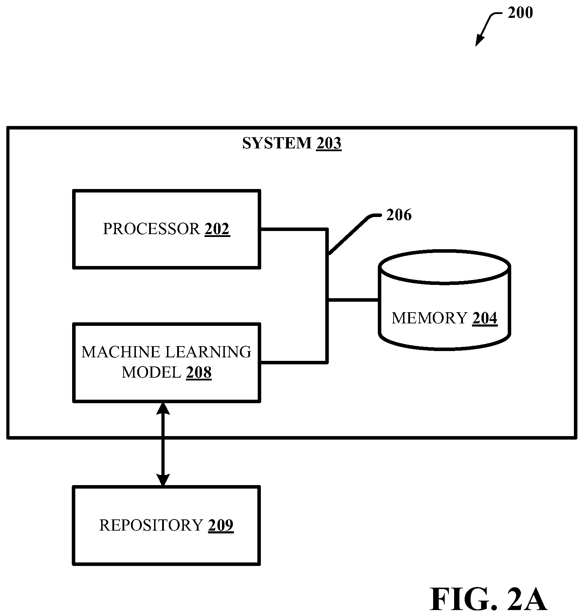

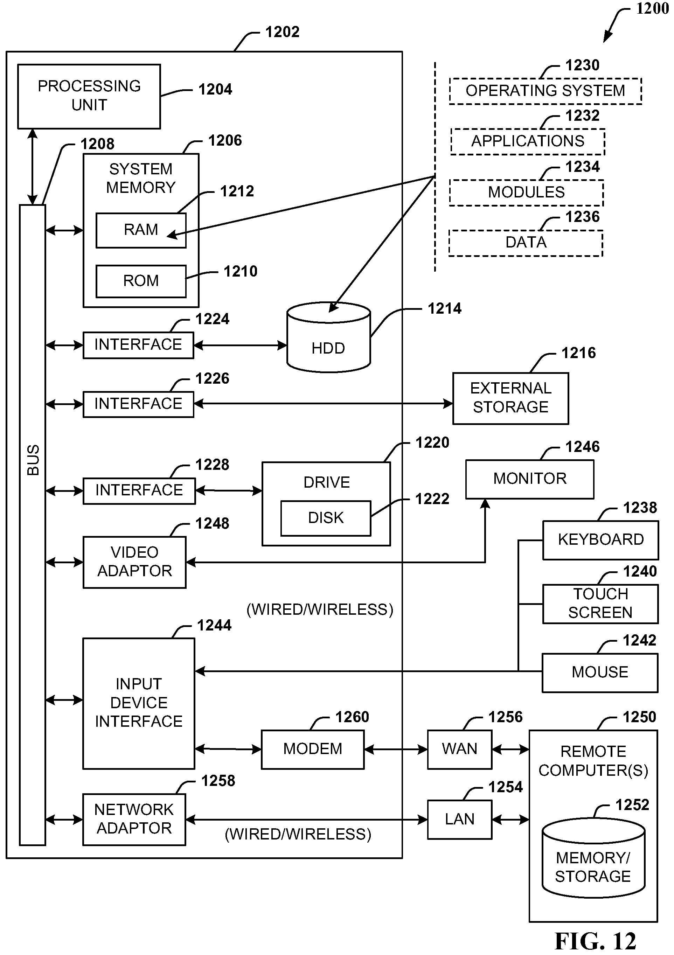

The embodiments depicted in one or more figures described herein are for illustration only, and as such, the architecture of embodiments is not limited to the systems, devices and/or components depicted therein, nor to any particular order, connection and/or coupling of systems, devices and/or components depicted therein. For example, in one or more embodiments, the non-limiting systems described herein, such as non-limiting system 200 as illustrated at FIG. 2A, and/or systems thereof, can further comprise, be associated with and/or be coupled to one or more computer and/or computing-based elements described herein with reference to an operating environment, such as the operating environment 1200 illustrated at FIG. 12. For example, non-limiting system 200 can be associated with, such as accessible via, a computing environment 1200 described below with reference to FIG. 12, such that aspects of processing can be distributed between non-limiting system 200 and the computing environment 1200. In one or more described embodiments, computer and/or computing-based elements can be used in connection with implementing one or more of the systems, devices, components and/or computer-implemented operations shown and/or described in connection with FIG.

2A and/or with other figures described herein.

FIG. 2A illustrates a block diagram of an example, non-limiting system 200 that can generate one or more AI models directed to different clinical protocols in accordance with one or more embodiments described herein.

Non-limiting system 200 and/or the components of non-limiting system 200 can be employed to use hardware and/or software to solve problems that are highly technical in nature (e.g., related to AI, federated learning, clinical protocols, etc.), that are not abstract and that cannot be performed as a set of mental acts by a human. Further, some of the processes performed may be performed by specialized computers for carrying out defined tasks related to a clinical protocol-based federated learning process. Non-limiting system 200 and/or components of non-limiting system 200 can be employed to solve new problems that arise through advancements in technologies mentioned above and/or the like. Non-limiting system 200 can provide technical improvements to machine learning systems and AI systems by reducing the time and effort involved in diagnosing medical conditions, increasing clinical accuracy of results generated by AI models, and increasing the speed of locally refining AI models in federated learning by reducing human-in-the-loop (HITL) efforts.

For example, traditional federated learning (as opposed to the embodiments disclosed herein) can be oversensitive or over specific leading to too many false positives or false negatives because the aggregated federated learning model (e.g., central AI model) does not account for custom clinical protocols followed by medical facilities. On the contrary, the embodiments disclosed herein employ multiple central AI models that are respectively directed to different respective clinical protocols, thereby reducing the number of false positive inferences and false negative inferences from the beginning of the process without waiting for the central AI models to learn and be fine tuned over time. In traditional federated learning, the time invested by clinicians (HITL time and effort) to relabel data for local use and to provide clinical feedback (e.g., accept/reject, thumbs up/thumbs down or a rating) is significantly large. The protocol-based federated learning process disclosed herein can generate more accurate outcomes for clinicals and hospitals while reducing the inferencing time and HITL efforts.

Discussion turns briefly to processor 202, memory 204 and bus 206 of non-limiting system 200. For example, in one or more embodiments, non-limiting system 200 can comprise processor 202 (e.g., computer processing unit, microprocessor, classical processor, and/or like processor). In one or more embodiments, a component associated with non-limiting system 200, as described herein with or without reference to the one or more figures of the one or more embodiments, can comprise one or more computer and/or machine readable, writable and/or executable components and/or instructions that can be executed by processor 202 to enable performance of one or more processes defined by such component(s) and/or instruction(s).

In one or more embodiments, non-limiting system 200 can comprise a computer-readable memory (e.g., memory 204) that can be operably connected to processor 202. Memory 204 can store computer-executable instructions that, upon execution by processor 202, can cause processor 202 and/or one or more other components of non-limiting system 200 (e.g., machine learning model 208, selection component 212, training component 214, deployment component 216 and/or storage component 218) to perform one or more actions. In one or more embodiments, memory 204 can store computer-executable components (e.g., machine learning model 208, selection component 212, training component 214, deployment component 216 and/or storage component 218).

Non-limiting system 200 and/or a component thereof as described herein, can be communicatively, electrically, operatively, optically and/or otherwise coupled to one another via bus 206. Bus 206 can comprise one or more of a memory bus, memory controller, peripheral bus, external bus, local bus, and/or another type of bus that can employ one or more bus architectures. One or more of these examples of bus 206 can be employed. In one or more embodiments, non-limiting system 200 can be coupled (e.g., communicatively, electrically, operatively, optically and/or like function) to one or more external systems (e.g., a non-illustrated electrical output production system, one or more output targets, an output target controller and/or the like), sources and/or devices (e.g., classical computing devices, communication devices and/or like devices), such as via a network. In one or more embodiments, one or more of the components of non-limiting system 200 can reside in the cloud, and/or can reside locally in a local computing environment (e.g., at a specified location(s)).

In various embodiments, non-limiting system 200 can represent a central server of a company or organization that can provide AI-based services. Non-limiting system 200 can comprise system 203 that can be a machine learning-based system that can be employed by the company or organization to provide the AI-based services. For example, system 203 can be employed in a protocol-based federated learning process to generate and maintain different AI models directed to different protocols followed by local sites or facilities, as opposed to a single central AI model. For example, system 203 can be employed to generate different AI models directed to different respective clinical protocols followed by different medical facilities such as hospitals, clinics, urgent care centers, trauma centers, etc. However, it should be appreciated that the embodiments herein are not limited to healthcare and can be applied to non-healthcare fields and non-clinical protocols.

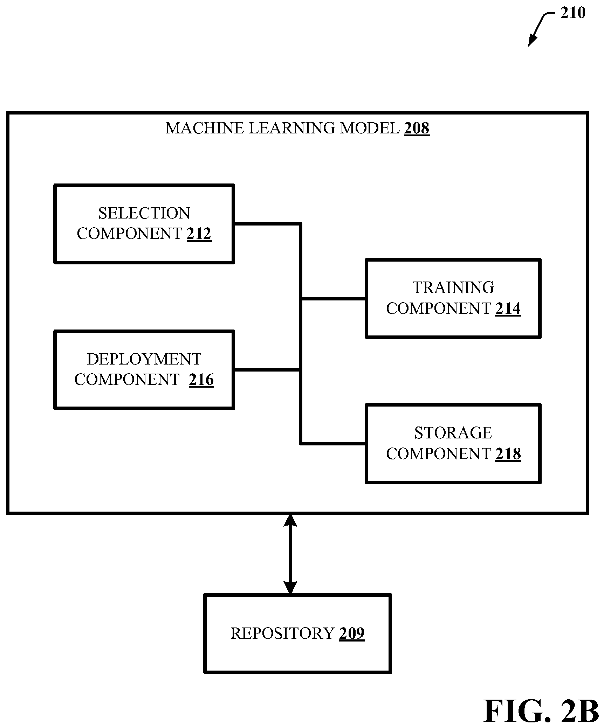

In various embodiments, system 203 can employ machine learning model 208 to generate and maintain the different AI models directed to the different respective clinical protocols. For example, machine learning model 208 can train a plurality of AI models (e.g., AI model C1, AI model C2, AI model C3, etc.). The plurality of AI models can be trained according to one or more respective clinical protocols (e.g., protocol 1 (P1), protocol 2 (P2), protocol 3 (P3), etc.), and the different AI models can be stored (e.g., via storage component 218 illustrated in FIG. 2B) in repository 209. Further, each AI model stored in repository 209 can be applicable to one or more medical facilities. For example, if two medical facilities follow the clinical protocol, P1, and AI model C1 is directed to P1, then machine learning model 208 can deploy AI model C1 at both medical facilities. If a third medical facility follows the clinical protocol, P2, and AI model, C2, is directed to P2, then machine learning model 208 can deploy AI model C2 at the third medical facility.

In one or more embodiments, machine learning model 208 can comprise selection component 212, training component 214, deployment component 216 and/or storage component 218, as illustrated in and further described with reference to FIG. 2B. Further, machine learning model 208 can interact with repository 209 to train and store the plurality of AI models in repository 209, wherein AI models stored in repository 209 can be accessed by machine learning model 208 and deployed to different facilities, such a hospitals or non-medical facilities, as part of a protocol-based federated learning process, via components (e.g., selection component 212, training component 214, deployment component 216 and/or storage component 218) comprised in machine learning model 208. In one or more embodiments, one or more machine learning models identical to machine learning model 208 can interact with repository 209 to train and store the plurality of AI models that can be deployed to different facilities such a hospitals or non-medical facilities as part of the protocol-based federated learning process.

In an embodiment, in addition to existing medical facilities serviced by machine learning model 208, at least one additional medical facility can sign up for the AI-based services provided by non-limiting system 200, and machine learning model 208 can select (e.g., via selection component 212 illustrated in FIG. 2B), according to a selection criterion applicable to the medical facility, a first AI model (e.g., an initial AI model) from repository 209, wherein repository 209 can comprise a plurality of AI models. Machine learning model 208 can deploy (e.g., via deployment component 216 illustrated in FIG. 2B) the first AI model at the medical facility. The first AI model can be employed to generate inferences on local data at the medical facility. An entity (e.g., hardware, software, neural network, AI, machine, and/or user) can assign labels (e.g., labels that identify correct/incorrect inferences) to assess the performance accuracy of the first AI model. In an embodiment, the entity can also generate clinical feedback (e.g., accept/reject, thumbs up/thumbs down or a rating) on the inferences. The first AI model can be retrained at the medical facility based on feedback comprising local data (e.g., patient data, clinical data, or any other type of data) generated at the medical facility, and the labels and clinical feedback generated by the entity. The training can generate a local AI model with updated parameters. Machine learning model 208 can access (e.g., after a defined time interval) the feedback comprising the updated parameters to select or generate a second AI model. Finally, machine learning model 208 can deploy the second AI model at the medical facility and continue to iteratively train the second AI model based on new feedback generated at the medical facility and/or one or more additional medical facilities via the same process.

In various embodiments, if the clinical protocol followed by the medical facility is known, selection component 212 can select an AI model directed to the clinical protocol as the first AI model, and deployment component 216 can deploy the first AI model at the medical facility. However, if the clinical protocol followed by the medical facility is unknown or not clearly defined/spelled out, selection component 212 can select the first AI model based on properties other than the clinical protocol. Such properties can comprise demographic information, size, geographical location, etc. associated with the medical facility, as described below. The clinical protocol for a medical facility can be unknown/undefined due to a non-standardization of protocols, for example, due to clinicians at the medical facility following varied protocols. In various embodiments, if the clinical protocol followed by the medical facility is known but repository 209 does not comprise an AI model directed to the clinical protocol, selection component 212 can select an AI model that is directed to a clinical protocol that is closest to the clinical protocol followed by the medical facility, as the first AI model. Finally, deployment component 216 can deploy the first AI model at the medical facility.

More specifically, in an embodiment, selection component 212 can employ a selection criterion wherein selection component 212 can compare one or more properties of the medical facility with one or more identical properties of the plurality of AI models, to select the first AI model. The one or more properties of the medical facility and the one or more identical properties of the plurality of AI models can be selected from a group consisting of a size of the medical facility, demographic information associated with the medical facility, a geographical location of the medical facility and the clinical protocol of the medical facility. In some embodiments, additional properties associated with the medical facility (e.g., demographic information, size, geographical location, etc.) can be considered, as described below. Such embodiments can be applicable whether the clinical protocol followed by the medical facility is known or unknown. For example, if the clinical protocol for the medical facility is known, selection component 212 can employ the clinical protocol as the property to select the first AI model, and if the clinical protocol for the medical facility is unknown, selection component 212 can employ properties other than the clinical protocol to select the first AI model. In some embodiments, selection component 212 can employ multiple properties such as the clinical protocol, demographic information, size, geographical location, etc. associated with the medical facility to select the first AI model.

In another embodiment, selection component 212 can employ a selection criterion wherein selection component 212 can analyze, according to a performance metric, respective performances of the plurality of AI models for the clinical protocol, to select the first AI model. Based on such analysis, selection component can select an AI model that has the best performance for the clinical protocol followed by the medical facility, as the first AI model. Deployment component 216 can deploy the first AI model at the medical facility for an initial round of inferencing. Such embodiments can be applicable when the clinical protocol followed by the medical facility is known but an AI model directed to the clinical protocol does not exist in repository 209.

As a result, the medical facility can initially receive an AI model (i.e., the first AI model) that can exhibit an accurate performance on the local data generated at medical facility or an AI model that can exhibit an acceptable performance on the local data generated at the medical facility. If the inferences generated by the first AI model deviate from the clinical protocol, the inferences can be labeled, and feedback can be generated for the first AI model. As noted *supra,* the feedback can comprise updated parameters generated as a result of training the first AI model on the training dataset as well as the labels and any clinical feedback assigned to the inferences. The updated parameters can comprise new weights and new gradients.

In an embodiment, the feedback can be accessed and employed by training component 214 (illustrated in FIG. 2B) to retrain the first AI model based on the updated parameters and the clinical protocol, if the first AI model is directed to the clinical protocol. For example, training component 214 can retrain the first AI model to update the weights and gradients of the first AI model based on the new weights and gradients comprised in the feedback. Such retraining can generate a new AI model that can be more accurate for the clinical protocol followed by the medical facility. In some implementations, if the first AI model is common to the medical facility and one or more additional medical facilities following the same protocol, training component 214 can aggregate the updated parameters generated at the medical facility and respective updated parameters generated at the one or more additional medical facilities to retrain the first AI model and generate the new AI model. Training component 214 can aggregate the updated parameters and the respective updated parameters based on an aggregation logic defined according to the clinical protocol. Deployment component 216 can deploy a second AI model (i.e., the new AI model) at the medical facility and the one or more additional medical facilities.

In another embodiment, the feedback can be accessed and employed by training component 214 to train an untrained AI model based on the updated parameters and the clinical protocol if the clinical protocol for the medical facility is known but an AI model directed to the clinical protocol does not exist in repository 209. For example, training component 214 can train an untrained AI model to generate a new AI model that is directed to the clinical protocol followed by the medical facility. In this regard, training component 214 can employ an AI model with an existing architecture and train the AI model on the feedback generated at the medical facility. In some implementations, if the first AI model is common to the medical facility and one or more additional medical facilities following the same protocol, training component 214 can aggregate the updated parameters generated at the medical facility and respective updated parameters generated at the one or more additional medical facilities to train the untrained AI model and generate the new AI model. Training component 214 can aggregate the updated parameters and the respective updated parameters based on an aggregation logic defined according to the clinical protocol employed by the medical facility and the one or more additional medical facilities. Deployment component 216 can deploy a second AI model (i.e., the new AI model) at the medical facility and/or one or more additional medical facilities.

In yet another embodiment, the feedback can be accessed and employed by selection component 212 to select an existing AI model from repository 209 if the clinical protocol for the medical facility was initially unknown. The existing AI model can be directed to the clinical protocol of the medical facility. For example, selection component 212 can compare the updated parameters of the first AI model with identical parameters of individual AI models of the plurality of AI models stored in repository 209. Based on the comparison, selection component 212 can select from repository 209, an existing AI model having parameters that are closest to the updated parameters (e.g., according to a defined metric), and deployment component 216 can deploy a second AI model (i.e., the existing AI model selected by selection component 212) to the medical facility.

In traditional federated learning, the feedback generated on the performance of an AI model at a medical facility is employed to train the AI model locally at the medical facility and the training can involve some human effort. By deploying the second AI model that can be directed to or more aligned with the protocol followed by a medical facility, the various embodiments herein can reduce such human effort by ensuring that the performance of the second AI model is at least as good as the first AI model, if not better than the first AI model. Further, deploying the second AI model based on the clinical protocol can increase accuracy and reduce a prediction time involved in predicting clinical outcomes and generating inferences based on the second AI model.

FIG. 2B illustrates a block diagram of an example, non-limiting system 210 that can generate one or more AI models directed to different clinical protocols in accordance with one or more embodiments described herein. Repetitive description of like elements and/or processes employed in respective embodiments is omitted for sake of brevity.

Non-limiting system 210 illustrates the system of machine learning model 208 and repository 209 described with reference to FIG. 2A. In an embodiment, machine learning model 208 can comprise selection component 212, training component 214, deployment component 216 and/or storage component 218. In another embodiment, machine learning model 208 can comprise additional or fewer components than illustrated in FIG. 2B. For example, training component 214 or another component can be located external to machine learning model 208 in system 203 or machine learning model 208 can comprise one or more additional non-illustrated components that can perform one or more of the operations described herein. Further, machine learning model 208 can interact with repository 209 to train and store AI models that can be accessed by machine learning model 208 via components comprised in machine learning model 208 and deployed to different facilities such a hospitals or non-medical facilities as part of a protocol-based federated learning process. In one or more embodiments, one or more machine learning models identical to machine learning model 208 can interact with repository 209 to train and store AI models that can be deployed to different facilities such a hospitals or non-medical facilities as part of a protocol-based federated learning process.

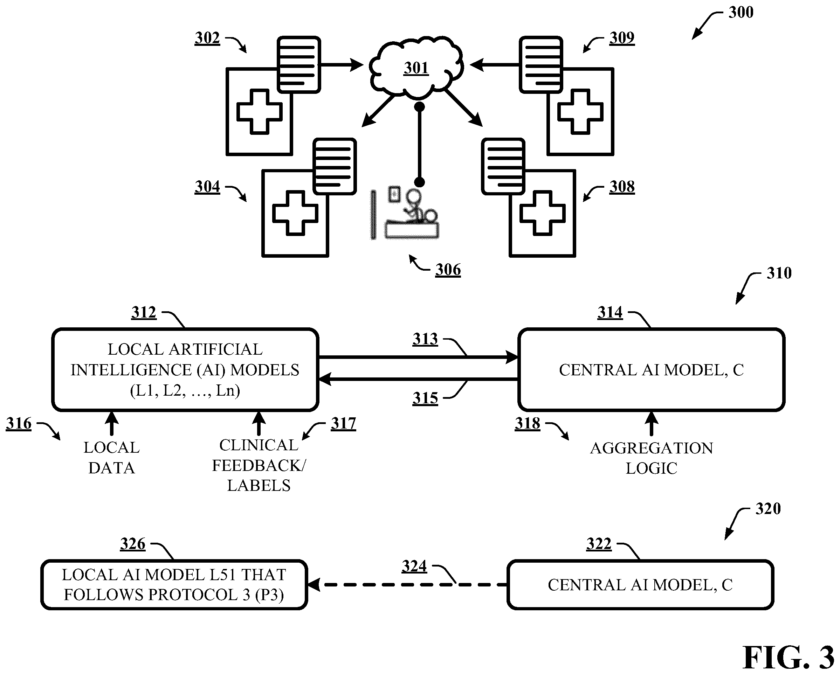

FIG. 3 illustrates diagrams of example, non-limiting methods 300, 310 and 320 applicable to federated learning. Non-limiting methods 300, 310 and 320 are intended to illustrate the processes involved in traditional federated learning and the problems associated with such processes in contrast to the embodiments of the present disclosure.

Non-limiting method 300 illustrates a process of federated learning that can be applicable to generate AI models directed to different clinical protocols. With the advent of AI technology in medical devices as part of clinical workflows at hospitals and medical sites, real-time feedback can be captured from the field to fine-tune AI models via federated learning. In general, federated learning can allow AI models to be trained across decentralized devices and/or sites while keeping data localized to the decentralized devices and/or sites due to privacy concerns. For example, instead of sending raw data to a central server, the central server can deploy an AI model to each decentralized device where the AI model can learn from local data.

Model updates, typically in the form of gradients and weights, can be shared with the central server and the central server can aggregate the model updates to improve a central AI model (also known as global or aggregated AI model). For example, in non-limiting method 300 numerals 302, 304, 306, 308 and 309 can represent individual medical facilities such as hospitals, clinics, etc. Hospital 302, hospital 308 and hospital 309 can share local updates with central server 301 (illustrated as a cloud), central server 301 can update an existing central AI model based on the local updates to generate a new central AI model, and central server 301 can share the new central AI model with hospital 304 by sharing aggregated weights and gradients of the new central AI model based on the local updates, wherein hospital 304 can be a new hospital that signs-up for the services of central server 301. Personal healthcare facility 306 can similarly receive the aggregated weights and gradients. Thereafter, hospital 304 and personal healthcare facility 306 can also share local updates with central server 301 after locally training the new central AI model, and the new AI model can be further updated. Thus, federated learning can enable collaborative learning without compromising data privacy because federated learning can involve aggregated learning of a central AI model at a central server based on model updates received by the central server from local sites.

The weights and gradients of the central AI model can be updated based on the aggregated learning, and the updated weights and gradients can be shared with the local sites. This is further illustrated by non-limiting method 310. For example, the central server can initially deploy central AI model C, to respective local sites. Each local site can employ central AI model C to generate inferences on local data at the respective local sites. An entity at each local site can generate labels for the inferences generated by central AI model C at the local site, based on the accuracies of the inferences. For example, a clinic can employ central AI model C to generate inferences on local data (e.g., patient data, etc.) generated at the clinic, and an entity at the clinical can generate labels for the inferences. In some embodiments, the entity can be a hardware, software, neural network, AI, and/or machine. In other embodiments, the entity can be a user, for example, an HITL. The entity can also generate additional clinical feedback for the inferences. The clinical feedback and labels are collectively identified as clinical feedback/labels 317 in FIG. 3.

At 312, the clinical feedback/labels 317 and local data 316 (e.g., patient data, etc.) from the clinic, can be employed to retrain central AI model C at the clinic, and retraining central AI model C can generate local AI model L1. As such, at 312, the respective local sites can generate respective local AI models L1, L2, ..., Ln. Retraining central AI model C can also generate updated weights and gradients. At 313, the clinic can transmit feedback comprising clinical feedback/labels 317 and the updated weights and gradients to the central server. The central server can access similar feedback from other local sites, and at 314, the central server can retrain central AI model C based on the feedback. The central server can employ aggregation logic 318 to retrain central AI model C. At 315, based on the retraining, the central server can send aggregated weights to the respective local sites and the process can continue.

AGGEREGATION LOGIC:

In machine learning, when training an AI model, the weights of the AI model are updated. For example, the weight of an AI model can be updated by $\Delta w1$, wherein w1 can represent the initial weight of the AI model and $\Delta w1$ can represent the value by which the weight can be updated. Similarly, the weight of another AI model can be updated by $\Delta w2$, wherein w2 can represent the initial weight of the AI model and $\Delta w2$ can represent the value by which the weight can be updated. In federated learning, a central AI model can be updated by $\dfrac{\Delta w1 + \Delta w2}{2}$. That is, the weights of at least two different local AI models can be aggregated to update the central AI model.

PROBLEMS WITH FEDERATED LEARNING:

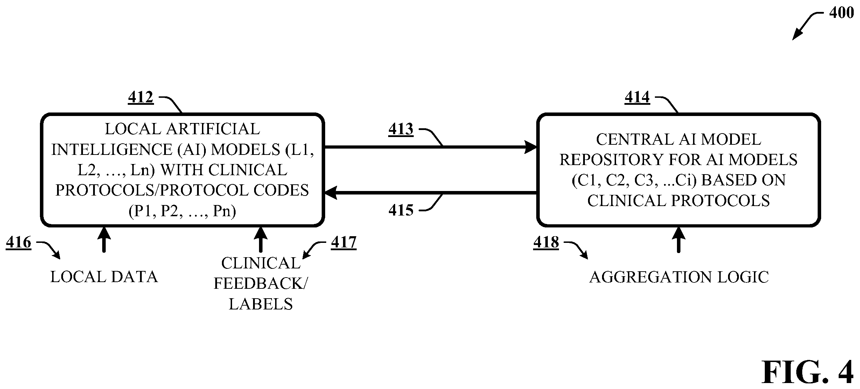

In clinical scenarios, the clinical protocols followed by hospitals and other local clinical sites can add to the complexity of federated learning because centrally aggregated trained models can reduce on-field performance due to variation in clinical protocols. For example, as illustrated by non-limiting method 320, at 322, central AI model C1, can be generated at the central server. Central AI model C1 can be trained by aggregating local data from 50 local sites, wherein each local site can follow protocol, P1, or protocol, P2. At 324, the central server can deploy central AI model C1 to a 51st local site that can be a newly enlisted site and that follows P3. At 326, the new local site can train central AI model C1 on local data generated at the new local site to further generate local AI model L51 directed to P3. However, as a result of central AI model C1 being trained on data for both P1 and P2, local AI model L51 can generate several false positives when employed to inference on the local data for the new local site. Further, local AI model L51 can be oversensitive and have low specificity owing to numerous false positives. This can further increase the need for an HITL and increase the clinical effort and time spent in relabeling and fine-tuning L51 for local use. On the contrary, the improved federated learning techniques described in embodiments of the present disclosure can avoid such problems by generating different AI models directed to different respective clinical protocols. This is explained in greater detail with reference to FIG. 4.

FIG. 4 illustrates a flow diagram of an example, non-limiting method 400 that can be employed as part of a protocol-based federated learning process in accordance with one or more embodiments described herein. Repetitive description of like elements and/or processes employed in respective embodiments is omitted for sake of brevity.

With continued reference to the embodiments of FIGs. 1 and 2, non-limiting method 400 illustrates a protocol-based federated learning process that can account for protocols followed by different local sites, such that a central server can generate and maintain different AI models directed to different respective protocols, as opposed to generating and maintaining a single central AI model. For example, at 415, a central server (e.g., non-limiting system 200) can deploy (e.g., via deployment component 216) central AI models (C1, C2, ..., Cn) to different respective local sites. The respective local sites can employ the central AI models to generate inferences on local data at the respective local sites. An entity at each local site can generate labels for the inferences generated by a central AI model at the local site, based on the accuracies of the inferences. For example, central AI model C1 can be deployed at hospital H1, and an entity at hospital H1 can label the inferences generate by central AI model C1 on local data at hospital H1. In some embodiments, the entity can be a hardware, software, neural network, AI, and/or machine. In other embodiments, the entity can be a user, for example, an HITL. The entity can also generate additional clinical feedback for the inferences. The clinical feedback and labels are collectively identified as clinical feedback/labels 417 in FIG. 4.

At 412, the clinical feedback/labels 417 and local data 416 (e.g., patient data, etc.) from the hospital, can be employed to retrain central AI model C1 at hospital H1, and retraining central AI model C1 can generate local AI model L1. As such, at 412, the respective local sites can generate respective local AI models L1, L2, ..., Ln directed to respective clinical protocols P1, P2, ..., Pn (or Pa, Pb, ... Pn). Retraining central AI model C1 can also generate updated weights and gradients for central AI model C1. At 413, the hospital can transmit feedback comprising clinical feedback/labels 417 and the updated weights and gradients to the central server. The central server can access similar feedback from other local sites, and at 414, the central server can retrain (e.g., via training component 214) different central AI models C1, C2, ..., Ci based on the feedback, as described with reference to FIG. 2A. Each central AI model C1, C2, ..., Ci, can be directed to a different clinical protocol (e.g., P1, P2, ..., Pn) and the central AI models can be saved in a central repository (e.g., repository 209).

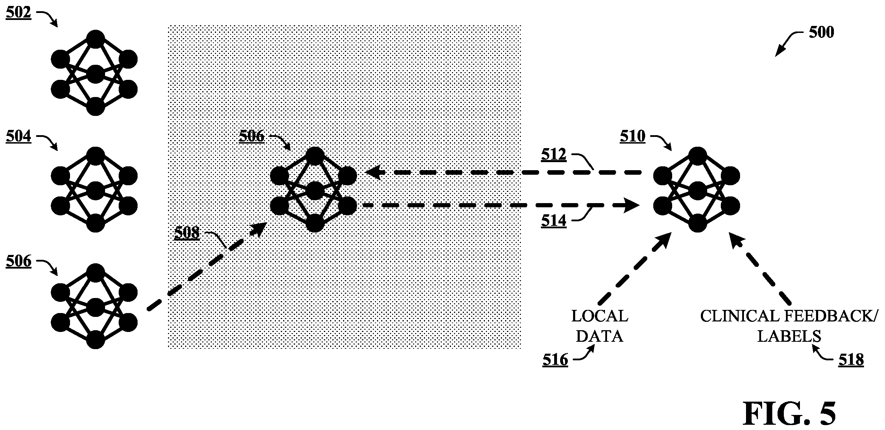

In some embodiments, as discussed with reference to FIG. 2A, if a central AI model directed to a clinical protocol does not exist in the central repository, the central server can train a new central AI model directed to that clinical protocol. These embodiments are further discussed with reference to FIG. 5. Further, the central server can employ aggregation logic 418 to train each central AI model. For example, if hospitals H1, H2 and H3 follow P1, and if central AI model C1 is directed to P1, the central server can employ aggregation logic 418 to retrain central AI model C1 based on feedback received from H1, H2 and H3, wherein the feedback can comprise clinical feedback, labels and updated weights and gradients generated for central AI model C1 at all three hospitals.

PROTOCOLS:

A clinical protocol followed by a medical facility can be a standard of clinical practice followed across the medical facility. Training a central AI model directed to a clinical protocol implies that the central AI model can generate inferences while accounting for the clinical protocol. For example, central AI model C1 can be an AI algorithm that can be trained to detect anomalies in cardiotocograph (CTG) data in labor and delivery (L & D) wards in hospitals. The AI algorithm can ingest fetal heart rate (FHR) data and uterine activity (UA) data to detect clinical events such as FHR accelerations, FHR decelerations and contractions. Further the AI algorithm can the detect clinical events according to clinical protocols. For example, clinicians follow clinical protocols that go beyond generally accepted standards. For example, FHR accelerations are events where the FHR rises above a baseline FHR value, and FHR accelerations can be classified according to clinical protocols followed by hospitals (e.g., as listed in Table 1), patient condition and history and any other factors relevant to the hospital (or another local site). Thus, referring to Table 1 shown below, if a hospital follows P2, an FHR can be classified as an acceleration at the hospital only if the FHR rises above a baseline FHR for the patient by at least 15 beats per minute (bpm) for a duration of at least 15 seconds.

Clinical protocols can also vary based on various other parameters. For example, in addition to clinical protocols customized to hospitals, clinical protocols can vary by geographic locations or demographics, and machine learning model 208 can generate customized AI models for clinical protocols employed in the United States of America (U.S.) versus clinical protocols employed in Europe (EU) or Asia. Clinical protocols can also vary by biological identity, and machine learning model 208 can generate customized AI models applicable to Caucasian people, Hispanic people, African American people, etc. In general, clinical protocols can vary according to a clinical context including history, regulations, geography, country specific standards, patient preferences, etc., and machine learning model 208 can generate different AI models directed to different respective clinical protocols regardless of the criteria by which the clinical protocols differ from one another.

Table 1:

| Protocol | Definition |
|---|---|
| Type A (Protocol 1 or P1) | 10 x 10 (rises above a baseline heart rate (HR) by at least 10 bpm and has a duration of at least 10 seconds) |
| Type B (Protocol 2 or P2) | 15 x 15 (rises above a baseline heart rate by at least 15 bpm and has a duration of at least 15 seconds) |
| Type C (Protocol 3 or P3) | 20 x 20 (rises above a baseline heart rate by at least 20 bpm and has a duration of at least 20 seconds) |

AGGREGATION LOGIC:

The aggregation logic employed by the central server can cater to the different clinical protocols. For example, properties such as demographics, clinical protocol, size, geographic locations, etc. of a first hospital can be 95% similar to the properties of a second hospital. In this scenario, a central AI model common to both hospitals can be trained (e.g., by training component 214) and maintained (e.g., in repository 209). As another example, if a central AI model is maintained for two hospitals, and three new hospitals having properties similar to the two hospitals sign up for the AI-based services of the central server, the central AI model can be updated based on feedback generate at all five hospitals, wherein the feedback can comprise labels assigned to inferences generated by respective local AI models employed by each of the five hospitals, updated weights and gradients generated for each local AI model as a result of locally training the local AI models, and any other feedback generate for the local AI models. Doing so can prevent the repository from becoming infinitely large and can conserve computational resources. Thus, if the central server services 100 hospitals, only 20 central AI models, for example, can be maintained in the repository.

FIG. 5 illustrates a flow diagram of an example, non-limiting method 500 that can be employed as part of a protocol-based federated learning process in accordance with one or more embodiments described herein. Repetitive description of like elements and/or processes employed in respective embodiments is omitted for sake of brevity.

With continued reference to the embodiments described with reference to FIGS. 1, 2 and 4, AI model 502 can represent central AI model C1 trained on local data from medical facilities that follow P1, AI model 504 can represent central AI model C2 trained on local data from medical facilities that follow P2, and AI model 506 can represent central AI model C3, trained on local data from medical facilities that follow P3. Machine learning model 208 can store (e.g., via storage component 218) AI models 502, 504 506 and one or more additional AI models in repository 209, and machine learning model 208 can perform (e.g., via training component 214) continuous and ongoing updates of individual AI models stored in repository 209.

In an embodiment, a new medical facility (e.g., hospital, clinical facility, etc.) can sign up for the AI-based services provided by machine learning model 208. If the new medical facility follows P3, then at 508, machine learning model 208 can initially select (e.g., via selection component 212) AI model 506 directed to P3, and at 514, machine learning model 208 can deploy (e.g., via deployment component 216) AI model 506 at the new medical facility. AI model 506 can be employed to generated inferences on local data at the new medical facility, and the inferences can be labeled by an entity (e.g., hardware, software, neural network, AI, machine and/or user). The entity can also provide additional clinical feedback for the inferences. In FIG. 5, the clinical feedback and labels are collectively identified as clinical feedback/labels 518. The new medical facility can retrain AI model 506 on local data 516 and clinical feedback/labels 518. The retraining can generate AI model 510 that can represent local AI model L3, and the retraining can generate updated weights and gradients for AI model 506. At 512, the new medical facility can share feedback comprising clinical feedback/labels 518 and the updated weights and gradients, with the central server, and the central server can retrain (e.g., via training component 214) AI model 506 based on the feedback.

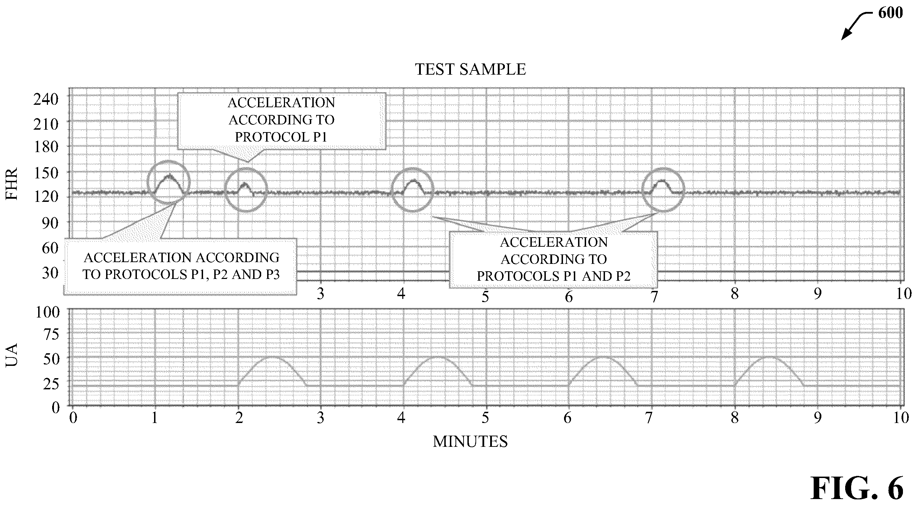

FIG. 6 illustrates a diagram of an example, non-limiting graph 600 that shows data that can be employed to train an AI model based on a clinical protocol in accordance with one or more embodiments described herein. Repetitive description of like elements and/or processes employed in respective embodiments is omitted for sake of brevity.

As described with reference to FIGS. 1, 2 and 4, a medical facility can follow a clinical protocol that can classify an FHR as an acceleration only if the FHR rises above a baseline FHR by at least a defined value and has a duration of a defined time interval. A protocol for a medical facility or a department in the medical facility can be determined by the head of the department or other concerned individuals to ensure consistency in the practices of all clinicians at the medical facility and/or at all sites associated with the medical facility.

When an AI model is trained for one clinical protocol, the AI model can become efficient at generating inferences based on the clinical protocol, and accordingly, the AI model can be validated and retrained for the clinical protocol. However, if an AI model is trained on multiple different clinical protocols, for example, on clinical protocols that contradict each other or define different threshold values for the same clinical parameter, the AI model can generate inferences based on an average of the different threshold values. That is, the inferences generated by the AI model can be based on an average of different threshold values defined by each clinical protocol that the AI model is trained for. Thus, by training different AI models for different clinical protocols, the various embodiments herein can ensure that each medical facility can receive a customized AI model that can be most efficient for the medical facility.

Non-limiting graph 600 illustrates fetal tracings with events. The waveform in the top half of non-limiting graph 600 illustrates FHR tracings and the peaks illustrate FHR accelerations (i.e., rises in the FHR). The waveform in the bottom half of non-limiting graph 600 illustrates UA tracings. In non-limiting graph 600, the FHR accelerations appear as peaks in the waveform, and an AI model can be trained to detect such FHR accelerations. For example, the height of each peak along the Y-axis of non-limiting graph 600 can represent the rise in the baseline FHR and the width of each peak along the X-axis of non-limiting graph 600 can represent the duration for which the FHR remains higher than the baseline. Training an AI model on a clinical protocol can imply that the AI model can ingest data, such as the waveform data illustrated by non-limiting graph 600, to detect events such as FHR accelerations, FHR decelerations and labor related contractions. For example, the AI model can detect and label one or more peaks in the waveform data as FHR accelerations according to the clinical protocol that the AI model is directed to. For example, in non-limiting graph 600, all four peaks in the FHR tracings data can represent FHRs that are higher than the baseline FHR by at least 10 bpm and have a duration of at least 10 seconds (P1). However, only the third and fourth peaks represent FHRs that are higher than the baseline FHR by at least 15 bpm and have a duration of at least 15 seconds (P2). Similarly, only the first peak represents an FHR that is higher than the baseline FHR by at least 20 bpm and has a duration of at least 20 seconds (P3). Thus, with reference to Table 1, only the first peak can be detected as an FHR acceleration by an AI model trained according to P3, only the third and fourth peaks can be detected as FHR accelerations by an AI model trained according to P2, and all four peaks can be detected as FHR accelerations by an AI model trained according to P1. The same concept can be applied to peaks in the UA data illustrated by non-limiting graph 600. As such, protocols can vary by sensitivity.

If an AI model directed to P1 (15 x 15) is deployed at a medical facility following P3, in practice, the AI model can generate three false positives (e.g., for the second, third and fourth peaks) for the waveform data illustrated by non-limiting graph 600. That is, the AI model can detect all four peaks as FHR accelerations instead of detecting only the first peak as an FHR acceleration according to P3. This can indicate that the AI model is highly sensitive and can be expected to generate many false positive inferences in the future for the local data generated at the medical facility. A highly sensitive AI model can generate more false positives and a less sensitive AI model can generate more false negatives and reduce the precision of the inferences. It should be noted that although non-limiting graph 600 illustrates only one event, there can be several events (20, 30, etc. events) for each interpretation. Thus, repository 209 can comprise many AI models depending on the use cases.

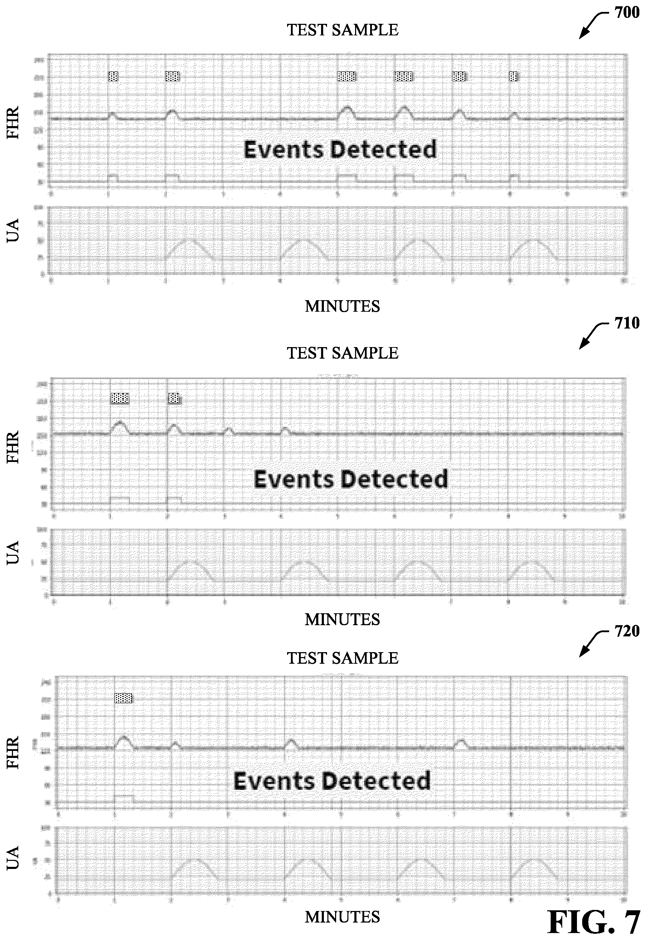

FIG. 7 illustrates diagrams of example, non-limiting graphs 700, 710 and 720 that show data that can be employed to train different AI models directed to different respective clinical protocols in accordance with one or more embodiments described herein. Repetitive description of like elements and/or processes employed in respective embodiments is omitted for sake of brevity.

In various embodiments, the waveform data from non-limiting graph 700 can be employed to train AI model C1 for hospitals that follow P1, the waveform data from non-limiting graph 710 can be employed to train AI model C2 for hospitals that follow P2, and the waveform data from non-limiting graph 720 can be employed to train AI model C3 for hospitals that follow P3, as discussed in greater detail with reference to Table 2 below. In this regard, non-limiting graph 700 illustrates annotated data directed to P1, non-limiting graph 710 illustrates annotated data directed to P2, and non-limiting graph 720 illustrates annotated data directed to P3.

As described with reference to at least FIG. 2A, in an embodiment, selection component 212 of machine learning model 208 can select, according to a selection criterion applicable to a medical facility, an AI model from repository 209, wherein repository 209 can comprise a plurality of AI models. The AI model can be deployed at the medical facility as part of an improved federated learning technique.

In some embodiments, selection component 212 can employ a selection criterion wherein selection component 212 can compare one or more properties of the medical facility with one or more identical properties of each AI model of a plurality of AI models stored in repository 209, to select the first AI model. The one or more properties of the medical facility and the one or more identical properties of the plurality of AI models can be selected from a group consisting of a size of the medical facility, demographic information associated with the medical facility, a geographical location of the medical facility and the clinical protocol of the medical facility. In some embodiments, additional properties associated with the medical facility can be considered. For example, machine learning model 208 can employ a clinical protocol, demographics, geographical location, ethnicity, etc. associated with the medical facility to select the AI model to be deployed at a medical facility.

In an embodiment, each AI model stored in repository 209 can be tagged (e.g., by storage component 218) for the different properties. For example, storage component 218 can tag each AI model with the clinical protocol that the AI model can be trained for, the geographic locations of hospitals that follow the clinical protocol, and/or any other applicable properties. Further, selection component 212 can identify the appropriate AI model for the medical facility by matching properties such as the clinical protocol, geographic location, and/or other applicable properties associated with the medical facility with the tags assigned to each AI model.

In another embodiment, storage component 218 can create a property file for each AI model. The property file for an AI model can comprise information about different characteristics associated with the AI model. For example, the property file can identify one or more ethnicities, populations of ethnicities, demographic diversity, clinical protocol, and/or other properties associated with the data employed to train the AI model. When a medical facility signs up for the AI-based service provided by machine learning model 208, selection component 212 can acquire information about the ethnicities, populations of ethnicities, demographic diversity, clinical protocol, etc. associated with the medical facility. Selection component 212 can compare the information associated with the medical facility to that of each AI model in repository 209 by employing the property table for each AI model. For example, repository 209 can comprise ten AI models and each AI model can be associated with a property file specific to the AI model. To select an initial AI model for a medical facility, selection component 212 can access a property file for the medical facility, wherein the property file can comprise the demographic information, clinical protocol information, geographic location information, etc. specific to the medical facility. Selection component 212 can compare the information in the property file for the medical facility with the information comprised in each of the ten property files for the ten AI models to identify the closest vectors. Based on the comparison, selection component 212 can select the AI model that is most applicable to the medical facility.

In some embodiments, selection component 212 can employ a selection criterion wherein selection component 212 can analyze, according to a performance metric, respective performances of the plurality of AI models stored in repository 209 for the clinical protocol followed by the medical facility to select an initial AI model for the medical facility. Based on such analysis, selection component 212 can select an AI model that has the best performance for the clinical protocol followed by the medical facility, as the first AI model. For example, selection component 212 can select an initial AI model for a medical facility based on an Area Under the Curve (AUC) metric. For example, an AI model trained on a clinical protocol can be labelled with the clinical protocol and with AUC values to identify the performance of the AI model on the clinical protocol and/or similar clinical protocols, and selection component 212 can analyze the labels to select the initial AI model. AUC is a metric that defines the performance of an AI model, and a higher AUC value of an AI model for a clinical protocol can indicate that the AI model can exhibit a better performance during inferencing (e.g., detection of medical conditions) for that clinical protocol. In an embodiment, metrics other than AUC can also be considered for the model selection.

AUC:

Considering clinical protocols P1, P2 and P3 listed in Table 1, when an AI model directed to P1 is deployed at a hospital that follows P2 or P3, the performance of the AI model can be sub-par at generating inferences on the local data generated at the hospital, as described elsewhere herein. On the contrary, an AI model directed to the same clinical protocol as that followed by the hospital can generate the best/highest AUC value. The highest AUC value for an AI model can be 1.000. However, if an AI model with a different clinical protocol is deployed at the hospital, the AI model can generate an AUC < 1.000. To determine the best possible AI model that can be deployed at the medical facility, selection component 212 can consider the respective AUC values for each AI model stored in repository 209. For example, as listed in Table 2, AI model C1 can be an AI model directed to P1, AI model C2 can be an AI model directed to P2, and AI model C3 can be an AI model directed to protocol P3. Selection component 212 can be trained to select an AI model with the highest possible AUC based on the clinical protocol followed by the medical facility. Thus, if the medical facility follows P3, selection component 212 can check repository 209 for an AI model having an AUC value of 1.000. For P3, the best AI model that can be initially deployed at the medical facility can be AI model C3. If repository 209 does not comprise AI model C3, selection component 212 can check repository 209 for the AI model with the next highest AUC value. For P3, the AI model with the next highest AUC value can be AI model C2. Thus, labels, property tables or AUC can be employed in the various embodiments to select an AI model.

Table 2: Experimental data based on AUC values

|  | AUC on P3 data | on P2 data | AUC on P1 data |
|---|---|---|---|
| AI model C3 | **1.000** | 0.665 | 0.571 |
| AI model C2 | 0.986 | **1.000** | 0.882 |
| AI model C1 | 0.980 | 0.993 | **1.000** |

The values in Table 2 are based on an experiment that was conducted to assess the performance of an AI model trained on a clinical protocol. It was observed, based on the AUC values, that:

If a medical facility follows P1, the order or preference during model selection can be C1 > C2 > C3.

If a medical facility follows P2, the order or preference during model selection can be C2 > C1 > C3.

If a medical facility follows P3, the order or preference during model selection can be C3 > C2 > C1.

If the clinical protocol followed by the medical facility is unknown, selection component 212 can select an appropriate initial AI model based on the applicable selection criteria (e.g., by comparing properties or tags associated with AI models stored in repository 209 with properties associated with the medical facility). Thereafter, selection component 212 can select a different AI model, or training component 214 can retrain the initial AI model or train a new AI model based on the labels, clinical feedback and/or updated weights and gradients generated for the initial AI model at the medical facility, as described elsewhere herein.

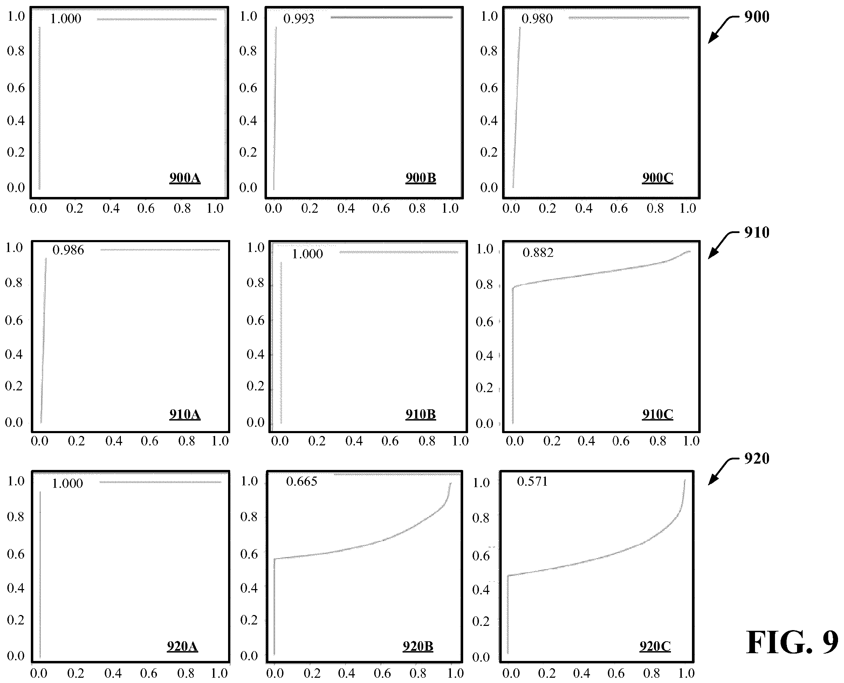

To determine the AUC for an AI model for different clinical protocols, the AI model can be trained, and the performance of the AI model can be analyzed for different clinical protocols. For example, an AI model can be trained on P1, and the performance of the AI model can be analyzed for P1, P2 and P3. The AUC curves for AI models C1, C2 and C3 for the experimental data listed in Table 1 are illustrated in FIG. 9. Training component 214 can employ a deep learning algorithm/methodology to train an AI model for a clinical protocol, and the training data employed to train the AI model can comprise different types of information. Continuing with the example previously discussed in this specification, in an embodiment, the training data to train an AI model to detect FHR accelerations for a certain clinical protocol can comprise waveform data showing FHR tracings, and the training data can be acquired from medical facilities that follow the clinical protocol. For example, the waveform data from non-limiting graph 700 can be employed to train AI model C1 for hospitals that follow P1, the waveform data from non-limiting graph 710 can be employed to train AI model C2 for hospitals that follow P2, and the waveform data from non-limiting graph 720 can be employed to train AI model C3 for hospitals that follow P3.

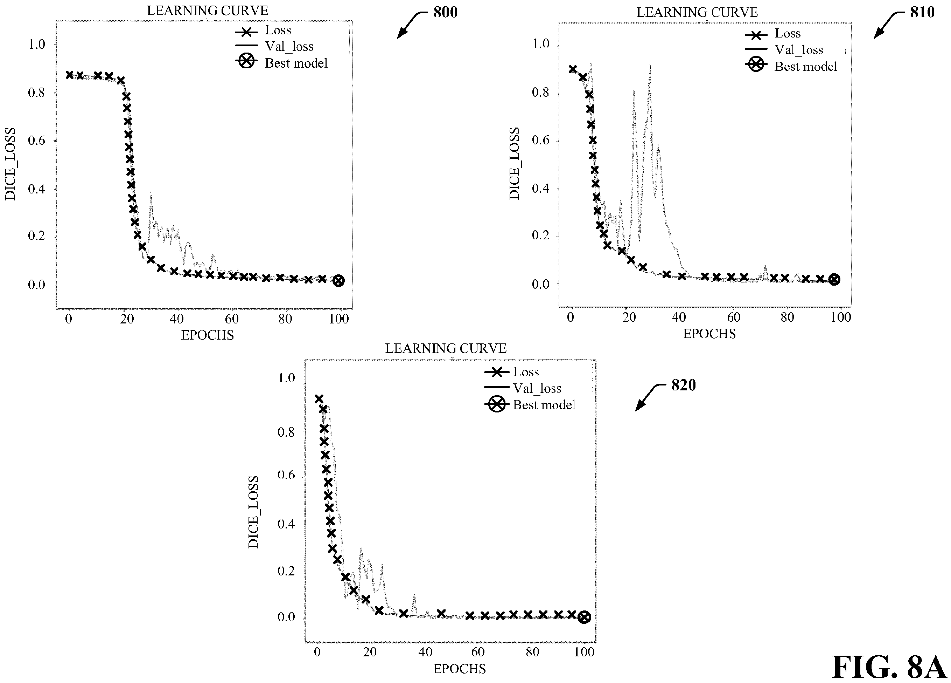

Further, the training data can comprise annotated data. For example, the peaks in the waveform data of non-limiting graphs 700, 710 and 720 can be annotated by an entity (e.g., hardware, software, neural network, AI, machine and/or user) to mark the FHR accelerations according to the clinical protocols applicable to each graph. In each of non-limiting graphs 700, 710 and 720, only the peaks that classify as FHR accelerations can be annotated by the entity. In FIG. 7, such annotations are illustrated by horizontal patterned blocks in each graph. Thus, to train an AI model for a clinical protocol, training data with annotations can be acquired from a hospital that follows the clinical protocol, wherein the annotations can indicate the FHR values that the AI model can be trained to detect as FHR accelerations. Thus, an AI model can be directed to a specific clinical protocol via annotations on training data. Additional details about training an AI model for a clinical protocol are described with reference to FIG. 8A.

FIG. 8A illustrates diagrams of example, non-limiting graphs 800, 810 and 820 that show training curves for different AI model directed to different respective clinical protocols in accordance with one or more embodiments described herein. Repetitive description of like elements and/or processes employed in respective embodiments is omitted for sake of brevity.

In deep learning, an input (x value) is given to a model, and the input can correspond to an expected output (y value). When a model is trained, the model is expected to generate a value y1 for an input value x1. However, in practice, the model generates a value y1 + $\Delta$y1, wherein the $\Delta$y1 indicates a difference or loss in the expected value versus the actual value generated by the model. Based on the difference, the model updates its parameters to fit into the expected value. In other words, the model starts learning on the training data. With continued reference to the embodiments discussed with reference to at least FIG. 7A, non-limiting graphs 800, 810 and 820 illustrate respective learning curves over iterations (number of epochs) for AI models, C1, C2 and C3, and non-limiting graphs 800, 810 and 820 show that the loss (i.e., deviation of the actual value from the expected value) of an AI model starts decreasing during training. In this regard, non-limiting graph 800 illustrates an AI model training curve for AI model C1 that can be trained on training data from a medical facility that follows P1, non-limiting graph 810 illustrates an AI model training curve for AI model C2 that can be trained on training data from a medical facility that follows P2, and non-limiting graph 820 illustrates an AI model training curve for AI model C1 that can be trained on training data from a medical facility that follows P3.

In general, during model training in machine learning, multiple types of losses can be considered such as, for example, dice loss, validation loss, etc. Dice loss (dice_loss in FIG. 8A) is a function that can generate an absolute difference between the expected value that the AI model can generate and the actual value generated by the AI model during model training, by applying the modulus function to the difference of the two values. Further, during the model training, a training set and a validation set can be employed to train the AI model. The training set can be input to the AI model and after training, the AI model can be tested on the validation set which can comprise data that is previously unseen by the AI model. The validation set is associated with a validation loss (val_loss in FIG. 8A) which is the equivalent of the dice loss for the validation set. Applying the trained AI model to the validation set ensures that the AI model has learnt correctly on the training set, and the validation loss is indicative that the AI model has been trained and that the AI model can generalize for unseen data.

In non-limiting graphs 800, 810 and 820, the curves with the 'x' symbols represent respective dice losses and the straight line curves represent the respective validation losses for AI models C1, C2 and C3, as illustrated by the legends inset in the graphs. In various embodiments herein, a similar training approach can be adopted by machine learning model 208 for initially training (e.g., via training component 214) one or more AI models based on different clinical protocols, and for retraining (e.g., via training component 214) an existing AI model based on updated weights and gradients for the AI model generated at a medical facility.

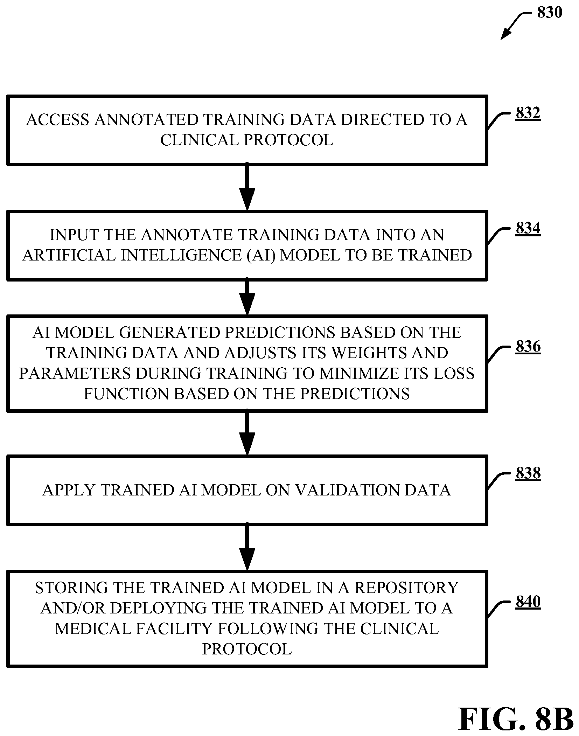

FIG. 8B illustrates a flow diagram of an example, non-limiting method 830 to train AI models directed to different respective clinical protocols in accordance with one or more embodiments described herein. Repetitive description of like elements and/or processes employed in respective embodiments is omitted for sake of brevity.

With continued reference to FIGS. 7 and 8A, non-limiting method 830 can be employed to train AI model C1 for protocol 1 (P1), AI model C2 for protocol 2 (P2), AI model C3 for protocol 3 (P3) or another AI model for a different protocol. For example, non-limiting method 830 can be employed to train AI model C1 by employing training data from non-limiting graph 700, and non-limiting graph 800 can illustrate the corresponding AI model training curve for AI model C1.

At 832, non-limiting method 830 can comprise accessing (e.g., by training component 214) annotated training data (e.g., non-limiting graphs 700, 710 or 720, or another type of annotated training data) directed to a clinical protocol (e.g., P1, P2, P3, etc.).

At 834, non-limiting method 830 can comprise inputting (e.g., by training component 214) the annotated training data into an AI model to be trained.

At 836, non-limiting method 830 can comprise generating (e.g., by the AI model) predictions based on the training data and adjusting (e.g., by the AI model) the weights and parameters of the AI model during training to minimize the loss function based on the predictions.

At 838, non-limiting method 830 can comprise validating (e.g., by training component 214) the AI model on validation data comprising data that is previously unseen by the AI model (e.g., FHR and UA data or other type of data) to assess the performance of the AI model.

At 840, non-limiting method 830 can comprise storing (e.g., by storage component 218) the trained AI model in a repository (e.g., repository 209) and/or deploying (e.g., by deployment component 216) the trained AI model at a medical facility following the clinical protocol.

FIG. 9 illustrates diagrams of example, non-limiting graph sets 900, 910 and 920 that show AUC curves for different AI models directed to different respective clinical protocols in accordance with one or more embodiments described herein. Repetitive description of like elements and/or processes employed in respective embodiments is omitted for sake of brevity.

With continued reference to the embodiments discussed with reference to at least FIGS. 7 and 8, non-limiting graph set 900 illustrates AUC curves of AI model C1 on test data for P1, non-limiting graph set 910 illustrates AUC curves of AI model C2 on test data for P2, and non-limiting graph set 920 illustrates AUC curves of AI model C3 on test data for P3. The test data in each scenario can be different from the training data employed to train each AI model. With reference to Tables 1 and 2, in non-limiting graph set 900, graph 900A illustrates the AUC curve for AI model C1 for P1, graph 900B illustrates the AUC curve for AI model C1 for P2, and graph 900C illustrates the AUC curve for AI model C1 for P3. Similarly, in non-limiting graph set 910, graph 910A illustrates the AUC curve for AI model C2 for P3, graph 910B illustrates the AUC curve for AI model C2 for P2, and graph 910C illustrates the AUC curve for AI model C2 for P1. Finally, in non-limiting graph set 920, graph 920A illustrates the AUC curve for AI model C3 for P3, graph 920B illustrates the AUC curve for AI model C3 for P2, and graph 920C illustrates the AUC curve for AI model C3 for P1.

Non-limiting graph sets 900, 910 and 920 are intended to provide a graphical representation of the AUC values for AI models directed to different protocols. As described above, an AI model directed to the same clinical protocol as that followed by a medical facility can generate the best/highest AUC value on data from the medical facility. The highest AUC value for an AI model can be 1.000. However, if an AI model with a different clinical protocol is deployed at the medical facility, the AI model can generate an AUC < 1.000. To determine the AUC value for an AI model for different clinical protocols, the AI model can be trained, and the performance of the AI model can be analyzed for different clinical protocols. For example, an AI model can be trained on P1, and the performance of the AI model can be analyzed for P1, P2 and P3. In this regard, graph 900A illustrates the AUC curve and value resulting from applying AI model C1 (directed to P1) on new test data directed to P1, graph 900B illustrates the AUC curve and value resulting from applying AI model C1 on new test data directed to P2 and graph 900C illustrates the AUC curve and value resulting from applying AI model C1 on new test data directed to P3. As evident, graph 900A illustrates the maximum area under the curve with an AUC value of 1.000. The same concept can be applied to the individual graphs in non-limiting graph sets 910 and 920. For example, graph 910A illustrates the AUC curve and value resulting from applying AI model C2 (directed to P2) on new test data directed to P3, graph 910B illustrates the AUC curve and value resulting from applying AI model C2 on new test data directed to P2, and graph 910C illustrates the AUC curve and value resulting from applying AI model C2 on new test data directed to P1. Similarly, graph 920A illustrates the AUC curve and value resulting from applying AI model C3 (directed to P3) on new test data directed to P3, graph 920B illustrates the AUC curve and value resulting from applying AI model C3 on new test data directed to P2, and graph 920C illustrates the AUC curve and value resulting from applying AI model C3 on new test data directed to P1.

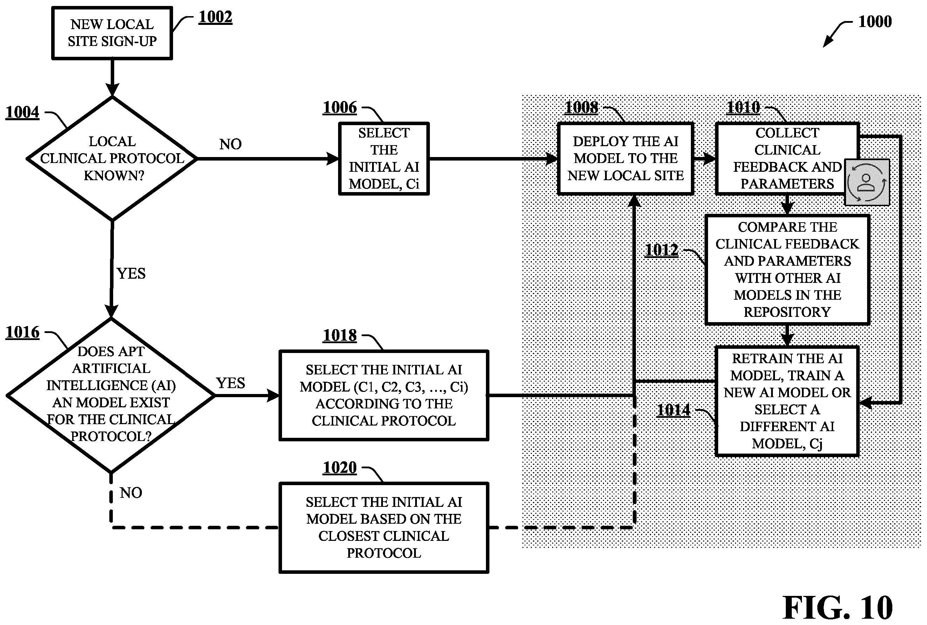

FIG. 10 illustrates a process flow diagram of an example, non-limiting pipeline 1000 that can be employed to generate one or more AI models directed to different clinical protocols in accordance with one or more embodiments described herein. Repetitive description of like elements and/or processes employed in respective embodiments is omitted for sake of brevity.

Non-limiting pipeline 1000 summarizes the protocol-based federated learning process described by embodiments of the present disclosure.

At 1002, a new local site (e.g., a medical facility such as a hospital, clinic, trauma center, etc.) can sign up for the AI-based services of machine learning model 208. The clinical protocol for the new local site can be known or unknown.

At 1004, machine learning model 208 can determine whether the clinical protocol is known or unknown.

If the clinical protocol is unknown or non-standardized, at 1006, selection component 212 can select an initial AI model, $C_i$, based on the applicable selection criteria. For example, selection component 212 can select $C_i$ by comparing properties or tags associated with AI models stored in repository 209 with the properties associated with the medical facility. In this regard, in some embodiments, a logic and method can be defined for selection of $C_i$. For example, $C_i$ can be based on a site, demography, location, etc. of the medical facility.

If the protocol is known, at 1016, selection component 212 can determine whether an apt AI model (i.e., an AI model directed to the clinical protocol) exists in repository 209.

If the apt AI model exists, at 1018, selection component 212 can select the initial AI model (e.g., C1, C2, ..., $C_i$) according to the clinical protocol.

If the AI model does not exist, at 1020, selection component 212 can select the initial AI model based on the closest clinical protocol (e.g., by analyzing the AUC values or other performance metric for the AI models stored in repository 209 for the clinical protocol followed by the new local site).

At 1008, deployment component 216 can deploy the initial AI model to the new local site.

At 1010, machine learning model 208 can access (e.g., via selection component 212 or training component 214) clinical feedback and updated parameters comprising new weights and new gradients generated for the initial AI model at the new local site as a result of training the initial AI model on local data and clinical feedback/labels generated for inferences made by the initial AI model at the new local site.

In an embodiment, training component 214 can retrain the initial AI model, at 1014, by updating the weights and gradients of the initial AI model based on the clinical feedback and the updated parameters. For example, if the initial AI model selected by selection component 212 (e.g., at 1018 in non-limiting pipeline 1000) is directed to the clinical protocol followed by the new local site, training component 214 can retrain the initial AI model based on the clinical feedback and the updated parameters to generate a new (updated) AI model, as opposed to selecting a different AI model from repository 209.

In another embodiment, selection component 212 can access the clinical feedback and the updated parameters generated for the initial AI model, and selection component 212 can compare, at 1012, the new weights and gradients for the initial AI model with the weights and gradients of existing AI models stored in repository 209. Based on the comparison, selection component 212 can identify a different AI model, $C_j$, having weights and gradients that are closest to the new weights and gradients of the initial AI model, and at 1014, selection component 212 can select $C_j$ to be deployed at the new local site.

In yet another embodiment, if an AI model with like parameters does not exist in repository 209, at 1014, training component 214 can train an untrained AI model based on the new weights and gradients.

At 1008, deployment component 216 can redeploy a second AI model (e.g., the retrained AI model, $C_j$, or the newly trained AI model) to the new local site. The cyclic process of ongoing continuous improvement of updated AI models including scenarios where the local clinical protocol is unknown or is ambiguous can continue to ensure that the AI model deployed at the new local site is ideal for the clinical protocol followed by the new local site.

Medical facilities such as hospitals can employ an HITL to ensure that the feedback (e.g., labels or other clinical feedback) documented for the inferences generated by the initial AI model is accurate. The HITL can edit/annotate the output of the AI model and new labels can be generated during the process. Based on the new labels, another local AI model can be trained according to the clinical protocol at the medical facility. During the training of the local AI model, new weights and gradients generated for the local AI model can be accessed by machine learning model 208 to update the initial AI model, select a different AI model, or train an untrained AI model, as opposed to training a central AI model that is common to different clinical protocols as in traditional federated learning.

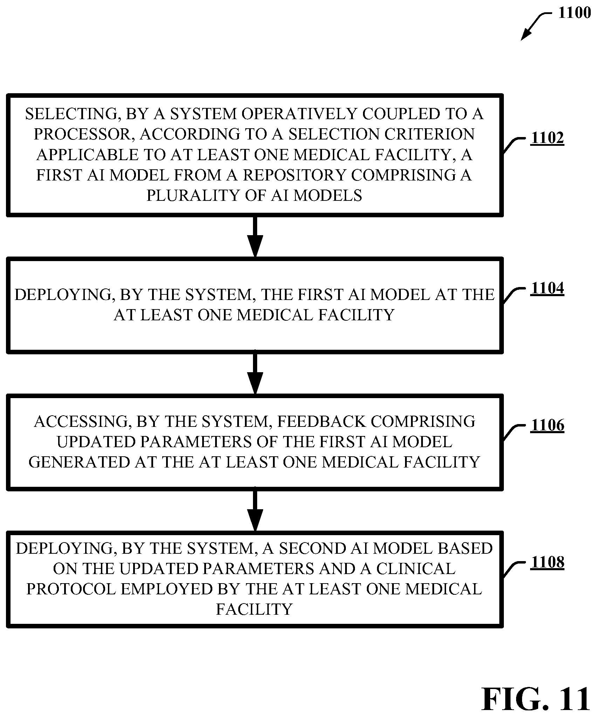

FIG. 11 illustrates a flow diagram of an example, non-limiting method 1100 that can generate one or more AI models directed to different clinical protocols in accordance with one or more embodiments described herein. Repetitive description of like elements and/or processes employed in respective embodiments is omitted for sake of brevity.

At 1102, the non-limiting method 1100 can comprise selecting (e.g., by selection component 212), by a system operatively coupled to a processor, according to a selection criterion applicable to at least one medical facility, a first AI model from a repository comprising a plurality of AI models.

At 1104, the non-limiting method 1100 can comprise deploying (e.g., by deployment component 216), by the system, the first AI model at the at least one medical facility.

At 1106, the non-limiting method 1100 can comprise accessing (e.g., by selection component 212, training component 214), by the system, feedback comprising updated parameters of the first AI model generated at the at least one medical facility.

At 1108, the non-limiting method 1100 can comprise deploying (e.g., by deployment component 216), by the system, a second AI model based on the updated parameters and a clinical protocol employed by the at least one medical facility.

For simplicity of explanation, the computer-implemented and non-computer-implemented methodologies provided herein are depicted and/or described as a series of acts. It is to be understood that the subject innovation is not limited by the acts illustrated and/or by the order of acts, for example acts can occur in one or more orders and/or concurrently, and with other acts not presented and described herein. Furthermore, not all illustrated acts can be utilized to implement the computer-implemented and non-computer-implemented methodologies in accordance with the described subject matter. Additionally, the computer-implemented methodologies described hereinafter and throughout this specification are capable of being stored on an article of manufacture to enable transporting and transferring the computer-implemented methodologies to computers. The term article of manufacture, as used herein, is intended to encompass a computer program accessible from any computer-readable device or storage media.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

In order to provide additional context for various embodiments described herein, FIG. 12 and the following discussion are intended to provide a brief, general description of a suitable computing environment 1200 in which the various embodiments described herein can be implemented. While the embodiments have been described above in the general context of computer-executable instructions that can run on one or more computers, those skilled in the art will recognize that the embodiments can be also implemented in combination with other program modules or as a combination of hardware and software.

Generally, program modules include routines, programs, components, data structures, etc., that perform particular tasks or implement particular abstract data types. Moreover, those skilled in the art will appreciate that the inventive methods can be practiced with other computer system configurations, including single-processor or multi-processor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can be also practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data or unstructured data.

Computer-readable storage media can include, but are not limited to, random access memory (RAM), read only memory (ROM), electrically erasable programmable read only memory (EEPROM), flash memory or other memory technology, compact disk read only memory (CD-ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible or non-transitory media which can be used to store desired information. In this regard, the terms "tangible" or "non-transitory" herein as applied to storage, memory or computer-readable media, are to be understood to exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

With reference again to FIG. 12, the example environment 1200 for implementing various embodiments of the aspects described herein includes a computer 1202, the computer 1202 including a processing unit 1204, a system memory 1206 and a system bus 1208. The system bus 1208 couples system components including, but not limited to, the system memory 1206 to the processing unit 1204. The processing unit 1204 can be any of various commercially available processors. Dual microprocessors and other multi-processor architectures can also be employed as the processing unit 1204.

The system bus 1208 can be any of several types of bus structure that can further interconnect to a memory bus (with or without a memory controller), a peripheral bus, and a local bus using any of a variety of commercially available bus architectures. The system memory 1206 includes ROM 1210 and RAM 1212. A basic input/output system (BIOS) can be stored in a non-volatile memory such as ROM, erasable programmable read only memory (EPROM), EEPROM, which BIOS contains the basic routines that help to transfer information between elements within the computer 1202, such as during startup. The RAM 1212 can also include a high-speed RAM such as static RAM for caching data.

The computer 1202 further includes an internal hard disk drive (HDD) 1214 (e.g., EIDE, SATA), one or more external storage devices 1216 (e.g., a magnetic floppy disk drive (FDD) 1216, a memory stick or flash drive reader, a memory card reader, etc.) and a drive 1220, e.g., such as a solid state drive, an optical disk drive, which can read or write from a disk 1222, such as a CD-ROM disc, a DVD, a BD, etc. Alternatively, where a solid state drive is involved, disk 1222 would not be included, unless separate. While the internal HDD 1214 is illustrated as located within the computer 1202, the internal HDD 1214 can also be configured for external use in a suitable chassis (not shown). Additionally, while not shown in environment 1200, a solid state drive (SSD) could be used in addition to, or in place of, an HDD 1214. The HDD 1214, external storage device(s) 1216 and drive 1220 can be connected to the system bus 1208 by an HDD interface 1224, an external storage interface 1226 and a drive interface 1228, respectively. The interface 1224 for external drive implementations can include at least one or both of Universal Serial Bus (USB) and Institute of Electrical and Electronics Engineers (IEEE) 1394 interface technologies. Other external drive connection technologies are within contemplation of the embodiments described herein.

The drives and their associated computer-readable storage media provide nonvolatile storage of data, data structures, computer-executable instructions, and so forth. For the computer 1202, the drives and storage media accommodate the storage of any data in a suitable digital format. Although the description of computer-readable storage media above refers to respective types of storage devices, it should be appreciated by those skilled in the art that other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 1212, including an operating system 1230, one or more application 1232, other program modules 1234 and program data 1236. All or portions of the operating system, applications, modules, or data can also be cached in the RAM 1212. The systems and methods described herein can be implemented utilizing various commercially available operating systems or combinations of operating systems.

Computer 1202 can optionally comprise emulation technologies. For example, a hypervisor (not shown) or other intermediary can emulate a hardware environment for operating system 1230, and the emulated hardware can optionally be different from the hardware illustrated in FIG. 12. In such an embodiment, operating system 1230 can comprise one virtual machine (VM) of multiple VMs hosted at computer 1202. Furthermore, operating system 1230 can provide runtime environments, such as the Java runtime environment or the .NET framework, for applications 1232. Runtime environments are consistent execution environments that allow applications 1232 to run on any operating system that includes the runtime environment. Similarly, operating system 1230 can support containers, and applications 1232 can be in the form of containers, which are lightweight, standalone, executable packages of software that include, e.g., code, runtime, system tools, system libraries and settings for an application.

Further, computer 1202 can be enabled with a security module, such as a trusted processing module (TPM). For instance, with a TPM, boot components hash next in time boot components, and wait for a match of results to secured values, before loading a next boot component. This process can take place at any layer in the code execution stack of computer 1202, e.g., applied at the application execution level or at the OS kernel level, thereby enabling security at any level of code execution.

A user can enter commands and information into the computer 1202 through one or more wired/wireless input devices, e.g., a keyboard 1238, a touch screen 1240, and a pointing device, such as a mouse 1242. Other input devices (not shown) can include a microphone, an infrared (IR) remote control, a radio frequency (RF) remote control, or other remote control, a joystick, a virtual reality controller or virtual reality headset, a game pad, a stylus pen, an image input device, e.g., camera(s), a gesture sensor input device, a vision movement sensor input device, an emotion or facial detection device, a biometric input device, e.g., fingerprint or iris scanner, or the like. These and other input devices are often connected to the processing unit 1204 through an input device interface 1244 that can be coupled to the system bus 1208, but can be connected by other interfaces, such as a parallel port, an IEEE 1394 serial port, a game port, a USB port, an IR interface, a BLUETOOTH® interface, etc.

A monitor 1246 or other type of display device can be also connected to the system bus 1208 via an interface, such as a video adapter 1248. In addition to the monitor 1246, a computer typically includes other peripheral output devices (not shown), such as speakers, printers, etc.

The computer 1202 can operate in a networked environment using logical connections via wired or wireless communications to one or more remote computers, such as a remote computer(s) 1250. The remote computer(s) 1250 can be a workstation, a server computer, a router, a personal computer, portable computer, microprocessor-based entertainment appliance, a peer device or other common network node, and typically includes many or all of the elements described relative to the computer 1202, although, for purposes of brevity, only a memory/storage device 1252 is illustrated. The logical connections depicted include wired/wireless connectivity to a local area network (LAN) 1254 or larger networks, e.g., a wide area network (WAN) 1256. Such LAN and WAN networking environments are commonplace in offices and companies, and facilitate enterprise-wide computer networks, such as intranets, all of which can connect to a global communications network, e.g., the Internet.

When used in a LAN networking environment, the computer 1202 can be connected to the local network 1254 through a wired or wireless communication network interface or adapter 1258. The adapter 1258 can facilitate wired or wireless communication to the LAN 1254, which can also include a wireless access point (AP) disposed thereon for communicating with the adapter 1258 in a wireless mode.

When used in a WAN networking environment, the computer 1202 can include a modem 1260 or can be connected to a communications server on the WAN 1256 via other means for establishing communications over the WAN 1256, such as by way of the Internet. The modem 1260, which can be internal or external and a wired or wireless device, can be connected to the system bus 1208 via the input device interface 1244. In a networked environment, program modules depicted relative to the computer 1202 or portions thereof, can be stored in the remote memory/storage device 1252. It will be appreciated that the network connections shown are examples and other means of establishing a communications link between the computers can be used.

When used in either a LAN or WAN networking environment, the computer 1202 can access cloud storage systems or other network-based storage systems in addition to, or in place of, external storage devices 1216 as described above, such as but not limited to a network virtual machine providing one or more aspects of storage or processing of information. Generally, a connection between the computer 1202 and a cloud storage system can be established over a LAN 1254 or WAN 1256 e.g., by the adapter 1258 or modem 1260, respectively. Upon connecting the computer 1202 to an associated cloud storage system, the external storage interface 1226 can, with the aid of the adapter 1258 or modem 1260, manage storage provided by the cloud storage system as it would other types of external storage. For instance, the external storage interface 1226 can be configured to provide access to cloud storage sources as if those sources were physically connected to the computer 1202.

The computer 1202 can be operable to communicate with any wireless devices or entities operatively disposed in wireless communication, e.g., a printer, scanner, desktop or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH® wireless technologies. Thus, the communication can be a predefined structure as with a conventional network or simply an ad hoc communication between at least two devices.

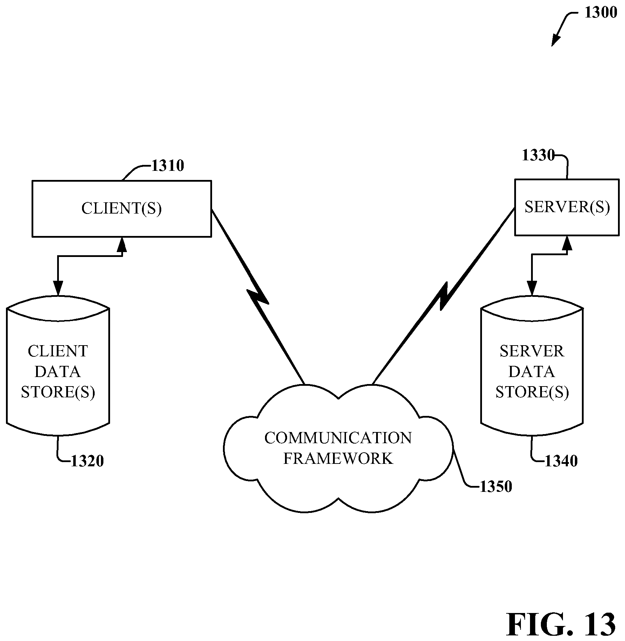

FIG. 13 is a schematic block diagram of a sample computing environment 1300 with which the disclosed subject matter can interact. The sample computing environment 1300 includes one or more client(s) 1310. The client(s) 1310 can be hardware or software (e.g., threads, processes, computing devices). The sample computing environment 1300 also includes one or more server(s) 1330. The server(s) 1330 can also be hardware or software (e.g., threads, processes, computing devices). The servers 1330 can house threads to perform transformations by employing one or more embodiments as described herein, for example. One possible communication between a client 1310 and a server 1330 can be in the form of a data packet adapted to be transmitted between two or more computer processes. The sample computing environment 1300 includes a communication framework 1350 that can be employed to facilitate communications between the client(s) 1310 and the server(s) 1330. The client(s) 1310 are operably connected to one or more client data store(s) 1320 that can be employed to store information local to the client(s) 1310. Similarly, the server(s) 1330 are operably connected to one or more server data store(s) 1340 that can be employed to store information local to the servers 1330.

Various embodiments may be a system, a method, an apparatus or a computer program product at any possible technical detail level of integration. The computer program product can include a computer readable storage medium (or media) having computer readable program instructions thereon for causing a processor to carry out aspects of various embodiments. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium can also include the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network or a wireless network. The network can comprise copper transmission cables, optical transmission fibers, wireless transmission, routers, firewalls, switches, gateway computers or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of various embodiments can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, including a local area network (LAN) or a wide area network (WAN), or the connection can be made to an external computer (for example, through the Internet using an Internet Service Provider). In some embodiments, electronic circuitry including, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform various aspects.

Various aspects are described herein with reference to flowchart illustrations or block diagrams of methods, apparatus (systems), and computer program products according to various embodiments. It will be understood that each block of the flowchart illustrations or block diagrams, and combinations of blocks in the flowchart illustrations or block diagrams, can be implemented by computer readable program instructions. These computer readable program instructions can be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart or block diagram block or blocks. These computer readable program instructions can also be stored in a computer readable storage medium that can direct a computer, a programmable data processing apparatus, or other devices to function in a particular manner, such that the computer readable storage medium having instructions stored therein comprises an article of manufacture including instructions which implement aspects of the function/act specified in the flowchart or block diagram block or blocks. The computer readable program instructions can also be loaded onto a computer, other programmable data processing apparatus, or other device to cause a series of operational acts to be performed on the computer, other programmable apparatus or other device to produce a computer implemented process, such that the instructions which execute on the computer, other programmable apparatus, or other device implement the functions/acts specified in the flowchart or block diagram block or blocks.

The flowcharts and block diagrams in the Figures illustrate the architecture, functionality, and operation of possible implementations of systems, methods, and computer program products according to various embodiments. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams or flowchart illustration, and combinations of blocks in the block diagrams or flowchart illustration, can be implemented by special purpose hardware-based systems that perform the specified functions or acts or carry out combinations of special purpose hardware and computer instructions.

While the subject matter has been described above in the general context of computer-executable instructions of a computer program product that runs on a computer or computers, those skilled in the art will recognize that this disclosure also can or can be implemented in combination with other program modules. Generally, program modules include routines, programs, components, data structures, etc. that perform particular tasks or implement particular abstract data types. Moreover, those skilled in the art will appreciate that various aspects can be practiced with other computer system configurations, including single-processor or multiprocessor computer systems, mini-computing devices, mainframe computers, as well as computers, hand-held computing devices (e.g., PDA, phone), microprocessor-based or programmable consumer or industrial electronics, and the like. The illustrated aspects can also be practiced in distributed computing environments in which tasks are performed by remote processing devices that are linked through a communications network. However, some, if not all aspects of this disclosure can be practiced on stand-alone computers. In a distributed computing environment, program modules can be located in both local and remote memory storage devices.

As used in this application, the terms "component," "system," "platform," "interface," and the like, can refer to or can include a computer-related entity or an entity related to an operational machine with one or more specific functionalities. The entities disclosed herein can be either hardware, a combination of hardware and software, software, or software in execution. For example, a component can be, but is not limited to being, a process running on a processor, a processor, an object, an executable, a thread of execution, a program, or a computer. By way of illustration, both an application running on a server and the server can be a component. One or more components can reside within a process or thread of execution and a component can be localized on one computer or distributed between two or more computers. In another example, respective components can execute from various computer readable media having various data structures stored thereon. The components can communicate via local or remote processes such as in accordance with a signal having one or more data packets (e.g., data from one component interacting with another component in a local system, distributed system, or across a network such as the Internet with other systems via the signal). As another example, a component can be an apparatus with specific functionality provided by mechanical parts operated by electric or electronic circuitry, which is operated by a software or firmware application executed by a processor. In such a case, the processor can be internal or external to the apparatus and can execute at least a part of the software or firmware application. As yet another example, a component can be an apparatus that provides specific functionality through electronic components without mechanical parts, wherein the electronic components can include a processor or other means to execute software or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. As used herein, the term "and/or" is intended to have the same meaning as "or." Moreover, articles "a" and "an" as used in the subject specification and annexed drawings should generally be construed to mean "one or more" unless specified otherwise or clear from context to be directed to a singular form. As used herein, the terms "example" or "exemplary" are utilized to mean serving as an example, instance, or illustration. For the avoidance of doubt, the subject matter disclosed herein is not limited by such examples. In addition, any aspect or design described herein as an "example" or "exemplary" is not necessarily to be construed as preferred or advantageous over other aspects or designs, nor is it meant to preclude equivalent exemplary structures and techniques known to those of ordinary skill in the art.

The herein disclosure describes non-limiting examples. For ease of description or explanation, various portions of the herein disclosure utilize the term "each," "every," or "all" when discussing various examples. Such usages of the term "each," "every," or "all" are non-limiting. In other words, when the herein disclosure provides a description that is applied to "each," "every," or "all" of some particular object or component, it should be understood that this is a non-limiting example, and it should be further understood that, in various other examples, it can be the case that such description applies to fewer than "each," "every," or "all" of that particular object or component.

As it is employed in the subject specification, the term "processor" can refer to substantially any computing processing unit or device comprising, but not limited to, single-core processors; single-processors with software multithread execution capability; multi-core processors; multi-core processors with software multithread execution capability; multi-core processors with hardware multithread technology; parallel platforms; and parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, or any combination thereof designed to perform the functions described herein. Further, processors can exploit nano-scale architectures such as, but not limited to, molecular and quantum-dot based transistors, switches and gates, in order to optimize space usage or enhance performance of user equipment. A processor can also be implemented as a combination of computing processing units. In this disclosure, terms such as "store," "storage," "data store," data storage," "database," and substantially any other information storage component relevant to operation and functionality of a component are utilized to refer to "memory components," entities embodied in a "memory," or components comprising a memory. It is to be appreciated that memory or memory components described herein can be either volatile memory or nonvolatile memory, or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory, or nonvolatile random access memory (RAM) (e.g., ferroelectric RAM (FeRAM). Volatile memory can include RAM, which can act as external cache memory, for example. By way of illustration and not limitation, RAM is available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM), and Rambus dynamic RAM (RDRAM). Additionally, the disclosed memory components of systems or computer-implemented methods herein are intended to include, without being limited to including, these and any other suitable types of memory.

What has been described above include mere examples of systems and computer-implemented methods. It is, of course, not possible to describe every conceivable combination of components or computer-implemented methods for purposes of describing this disclosure, but many further combinations and permutations of this disclosure are possible. Furthermore, to the extent that the terms "includes," "has," "possesses," and the like are used in the detailed description, claims, appendices and drawings such terms are intended to be inclusive in a manner similar to the term "comprising" as "comprising" is interpreted when employed as a transitional word in a claim.

The descriptions of the various embodiments have been presented for purposes of illustration, but are not intended to be exhaustive or limited to the embodiments disclosed. Many modifications and variations will be apparent without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

**Claims**

1. A system, comprising:

   a memory that stores computer executable components; and
   a processor that executes at least one of the computer executable components that:

      selects, according to a selection criterion applicable to at least one medical facility, a first artificial intelligence (AI) model from a repository comprising a plurality of AI models;
      deploys the first AI model at the at least one medical facility;
      accesses feedback comprising updated parameters of the first AI model generated at the at least one medical facility; and
      deploys a second AI model based on the updated parameters and a clinical protocol employed by the at least one medical facility.

2. The system of claim 1, wherein deploying the second AI model comprises:

   retraining the first AI model or training an untrained AI model based on the updated parameters and the clinical protocol;

generating a new AI model based on the retraining or the training; and

deploying the new AI model as the second AI model.

3. The system of claim 2, wherein the training or the retraining is based on an aggregation logic defined according to the clinical protocol employed by the at least one medical facility and one or more additional medical facilities.

4. The system of claim 1, wherein deploying the second AI model comprises:

comparing the updated parameters of the first AI model with identical parameters of individual AI models comprised in the plurality of AI models;

selecting an existing AI model from the repository based on the comparing; and

deploying the existing AI model as the second AI model.

5. The system of claim 1, wherein the plurality of AI models are trained according to one or more respective clinical protocols.

6. The system of claim 1, wherein the selection criterion comprises comparing one or more properties of the at least one medical facility with one or more identical properties of the plurality of AI models, wherein the one or more properties of the at least one medical facility and the one or more identical properties of the plurality of AI models are selected from a group consisting of a size of the at least one medical facility, demographic information associated with the at least one medical facility, a geographical location of the at least one medical facility and the clinical protocol.

7. The system of claim 1, wherein the selection criterion comprises analyzing, according to a performance metric, respective performances of the plurality of AI models for the clinical protocol.

8. The system of claim 1, wherein deploying the second AI model based on the clinical protocol increases accuracy and reduces a prediction time involved in predicting clinical outcomes based on the second AI model.

9. A computer-implemented method, comprising:

selecting, by a system operatively coupled to a processor, according to a selection criterion applicable to at least one medical facility, a first AI model from a repository comprising a plurality of AI models;

deploying, by the system, the first AI model at the at least one medical facility;

accessing, by the system, feedback comprising updated parameters of the first AI model generated at the at least one medical facility; and

deploying, by the system, a second AI model based on the updated parameters and a clinical protocol employed by the at least one medical facility.

10. The computer-implemented method of claim 9, wherein the deploying comprises:

retraining, by the system, the first AI model or training an untrained AI model based on the updated parameters and the clinical protocol;

generating, by the system, a new AI model based on the retraining or the training; and

deploying, by the system, the new AI model as the second AI model, and/or

wherein the training or the retraining is based on an aggregation logic defined according to the clinical protocol employed by the at least one medical facility and one or more additional medical facilities.

11. The computer-implemented method of claim 9, wherein the deploying comprises:

comparing, by the system, the updated parameters of the first AI model with identical parameters of individual AI models comprised in the plurality of AI models;

selecting, by the system, an existing AI model from the repository based on the comparing; and

deploying, by the system, the existing AI model as the second AI model.

12. The computer-implemented method of claim 9, wherein the plurality of AI models are trained according to one or more respective clinical protocols.

13. The computer-implemented method of claim 9, further comprising:

comparing, by the system, one or more properties of the at least one medical facility with one or more identical properties of the plurality of AI models, wherein the one or more properties of the at least one medical facility and the one or more identical properties of the plurality of AI models are selected from a group consisting of a size of the at least one medical facility, demographic information associated with the at least one medical facility, a geographical location of the at least one medical facility and the clinical protocol.

14. The computer-implemented method of claim 9, further comprising:
analyzing, by the system, according to a performance metric, respective performances of the plurality of AI models for the clinical protocol.

15. The computer-implemented method of claim 9, wherein deploying the second AI model based on the clinical protocol increases accuracy and reduces a prediction time involved in predicting clinical outcomes based on the second AI model.

MEDICAL FACILITY **A**

MEDICAL FACILITY **D**

MEDICAL FACILITY **B**

MEDICAL FACILITY **C**

**E**

100

**FIG. 1**

200

**SYSTEM 203**

PROCESSOR **202**

206

MEMORY **204**

MACHINE LEARNING
MODEL **208**

REPOSITORY **209**

# FIG. 2A

210

MACHINE LEARNING MODEL 208

SELECTION COMPONENT 212

TRAINING COMPONENT 214

DEPLOYMENT COMPONENT 216

STORAGE COMPONENT 218

REPOSITORY 209

**FIG. 2B**

FIG. 3

EP 4 664 487 A1

400

414 — CENTRAL AI MODEL REPOSITORY FOR AI MODELS (C1, C2, C3, ...Ci) BASED ON CLINICAL PROTOCOLS

418 — AGGREGATION LOGIC

413

415

412 — LOCAL ARTIFICIAL INTELLIGENCE (AI) MODELS (L1, L2, ..., Ln) WITH CLINICAL PROTOCOLS/PROTOCOL CODES (P1, P2, ..., Pn)

417 — CLINICAL FEEDBACK/ LABELS

416 — LOCAL DATA

**FIG. 4**

FIG. 5

EP 4 664 487 A1

FIG. 6

TEST SAMPLE

700

**FHR**

**Events Detected**

**UA**

MINUTES

710

TEST SAMPLE

**FHR**

**Events Detected**

**UA**

MINUTES

720

TEST SAMPLE

**FHR**

**Events Detected**

**UA**

MINUTES

**FIG. 7**

FIG. 8A

830

```
┌──────────────────────────────────────────────┐
│  ACCESS ANNOTATED TRAINING DATA DIRECTED TO A  │──  832
│             CLINICAL PROTOCOL                  │
└──────────────────────────────────────────────┘
                      │
                      ▼
┌──────────────────────────────────────────────┐
│     INPUT THE ANNOTATE TRAINING DATA INTO AN   │──  834
│  ARTIFICIAL INTELLIGENCE (AI) MODEL TO BE TRAINED │
└──────────────────────────────────────────────┘
                      │
                      ▼
┌──────────────────────────────────────────────┐
│   AI MODEL GENERATED PREDICTIONS BASED ON THE   │
│   TRAINING DATA AND ADJUSTS ITS WEIGHTS AND    │──  836
│ PARAMETERS DURING TRAINING TO MINIMIZE ITS LOSS │
│      FUNCTION BASED ON THE PREDICTIONS          │
└──────────────────────────────────────────────┘
                      │
                      ▼
┌──────────────────────────────────────────────┐
│    APPLY TRAINED AI MODEL ON VALIDATION DATA    │──  838
└──────────────────────────────────────────────┘
                      │
                      ▼
┌──────────────────────────────────────────────┐
│  STORING THE TRAINED AI MODEL IN A REPOSITORY   │
│  AND/OR DEPLOYING THE TRAINED AI MODEL TO A    │──  840
│   MEDICAL FACILITY FOLLOWING THE CLINICAL      │
│               PROTOCOL                          │
└──────────────────────────────────────────────┘
```

**FIG. 8B**

FIG. 9

**FIG. 10**

1100

SELECTING, BY A SYSTEM OPERATIVELY COUPLED TO A
PROCESSOR, ACCORDING TO A SELECTION CRITERION
APPLICABLE TO AT LEAST ONE MEDICAL FACILITY, A
FIRST AI MODEL FROM A REPOSITORY COMPRISING A
PLURALITY OF AI MODELS
— 1102

DEPLOYING, BY THE SYSTEM, THE FIRST AI MODEL AT THE
AT LEAST ONE MEDICAL FACILITY
— 1104

ACCESSING, BY THE SYSTEM, FEEDBACK COMPRISING
UPDATED PARAMETERS OF THE FIRST AI MODEL
GENERATED AT THE AT LEAST ONE MEDICAL FACILITY
— 1106

DEPLOYING, BY THE SYSTEM, A SECOND AI MODEL BASED
ON THE UPDATED PARAMETERS AND A CLINICAL
PROTOCOL EMPLOYED BY THE AT LEAST ONE MEDICAL
FACILITY
— 1108

FIG. 11

**FIG. 12**

1300

1310

CLIENT(S)

1330

SERVER(S)

CLIENT
DATA
STORE(S)

1320

COMMUNICATION
FRAMEWORK

1350

SERVER
DATA
STORE(S)

1340

**FIG. 13**

Europäisches
Patentamt

European
Patent Office

Office européen
des brevets

# EUROPEAN SEARCH REPORT

**Application Number**

EP 25 17 7169

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/316141 A1 (TOPOREK GRZEGORZ ANDRZEJ [US] ET AL) 5 October 2023 (2023-10-05) * paragraphs [0035] - [0114] * ----- | 1-15 | INV. G16H50/70 |
| X | WO 2022/261192 A1 (FREENOME HOLDINGS INC [US]) 15 December 2022 (2022-12-15) * paragraphs [0165] - [0361] * ----- | 1-15 | |
| A | DHADE PALLAVI ET AL: "Federated Learning for Healthcare: A Comprehensive Review", DEPARTMENT OF COMPUTER SCIENCE & ENGINEERING, SANDIP UNIVERSITY, NASHIK 422213, MAHARASHTRA, INDIA, 1 January 2023 (2023-01-01), page 230, XP093326119, DOI: 10.3390/engproc2023059230 * the whole document * ----- | 1-15 | |
| A | ZHANG FAN ET AL: "Recent methodological advances in federated learning for healthcare", PATTERNS, vol. 5, no. 6, 1 June 2024 (2024-06-01), page 101006, XP093326187, ISSN: 2666-3899, DOI: 10.1016/j.patter.2024.101006 * the whole document * ----- | 1-15 | **TECHNICAL FIELDS SEARCHED (IPC)** G16H |

The present search report has been drawn up for all claims

| Place of search | Date of completion of the search | Examiner |
|---|---|---|
| Munich | 20 October 2025 | Díaz de Lezana, C |

EPO FORM 1503 03.82 (P04C01)

## ANNEX TO THE EUROPEAN SEARCH REPORT
## ON EUROPEAN PATENT APPLICATION NO.

EP 25 17 7169

This annex lists the patent family members relating to the patent documents cited in the above-mentioned European search report.
The members are as contained in the European Patent Office EDP file on
The European Patent Office is in no way liable for these particulars which are merely given for the purpose of information.

20-10-2025

| Patent document cited in search report | Publication date | Patent family member(s) | Publication date |
|---|---|---|---|
| US 2023316141 A1 | 05-10-2023 | NONE | |
| WO 2022261192 A1 | 15-12-2022 | CA 3220786 A1 | 15-12-2022 |
| | | EP 4352745 A1 | 17-04-2024 |
| | | US 2024289586 A1 | 29-08-2024 |
| | | WO 2022261192 A1 | 15-12-2022 |

EPO FORM P0459

For more details about this annex : see Official Journal of the European Patent Office, No. 12/82